# EUROPEAN PATENT SPECIFICATION

(11) **EP 0 548 186 B1**
(45) Date of publication and mention of the grant of the patent: **05.03.1997**
(21) Application number: 91916416.0
(22) Date of filing: 09.09.1991
(51) Int. Cl.: C07D 501/36, C07D 501/59, C07D 501/20, C07D 501/18, A61K 31/545

(54) **CEPHALOSPORIN DERIVATIVES AND THEIR HOMOLOGUES**
CEPHALOSPORIN-DERIVATE UND DEREN HOMOLOGE
DERIVES DE CEPHALOSPORINE ET LEURS HOMOLOGUES

(30) Priority: 10.09.1990 GB 9019743
(43) Date of publication of application: 30.06.1993
(73) Proprietor: Beecham Group p.l.c., Brentford, Middlesex TW8 9BD (GB)
(72) Inventor: GASSON, B. C, SmithKline Beecham Pharmaceuticals, Surrey RH3 7AJ (GB); HINKS, J. D., SmithKline Beecham Pharmaceuticals, Surrey RH3 7AJ (GB); BURTON, George SmithKline Beecham Pharmaceuticals, Surrey RH3 7AJ (GB)
(74) Representative: Moore, James William
(86) International application number: GB9101534
(87) International publication number: WO9204353

(56) References cited:
- EP-A- 0 359 536
- EP-A- 0 395 219
- EP-A- 0 408 034
- EP-A- 0 418 020

## Description

This invention relates to novel β-lactam containing compounds, their preparation and their use, and in particular to a novel class of cephalosporins. These compounds have antibacterial properties, and therefore are of use in the treatment of bacterial infections in humans and animals caused by a wide range of organisms.

EP-A-0 359 536 (Beecham Group plc) discloses cephalosporin compounds of formula (A): 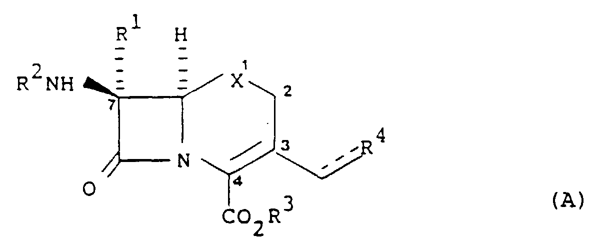 wherein R¹ is hydrogen, methoxy or formamido; R² is an acyl group; CO₂R³ is a carboxy group or a carboxylate anion, or R³ is a readily removable carboxy protecting group; R⁴ is an optionally substituted butenolide or butanolide ring; X is S, SO, SO₂, O or CH₂; and the dashed line adjacent to R⁴ represents an optional double band.

We have now found a particular class of cephalosporins bearing a novel 3-position substituent that possesses high antibacterial activity and also shows good parenteral and oral absorption.

The present invention provides a compound of formula (I) or a salt thereof: 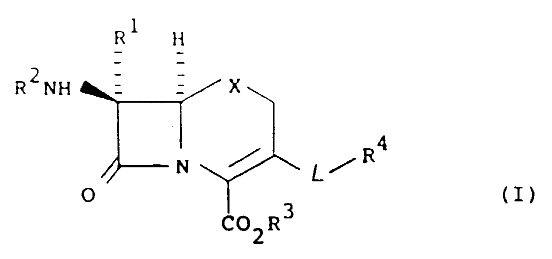 wherein
R¹ is hydrogen, methoxy or formamido;
R² is an acyl group, in particular that of an antibacterially active cephalosporin; CO₂R³ is a carboxy group or a carboxylate anion, or R³ is a readily removable carboxy protecting group (such as a pharmaceutically acceptable in vivo hydrolysable ester group);
L is -CH=,-(CH₂)ₙ where n is 0 or 1, -(CH₂)ₓ-Y-(CH₂)_{y}- where x and y are independently 0 or 1 and Y is sulphur or oxygen;
R⁴ is a γ- or δ-thiolactone or lactam ring optionally containing one or (where applicable) two endocyclic double bonds, which ring is optionally substituted at any carbon atom by alkyl, alkenyl, alkynyl, amino, acylamino, dialkylamino, alkoxy, hydroxy, halogen, carboxy, C₁₋₆ alkoxycarbonyl, amido, di-(C₁₋₆)alkylamido, di-(C₁₋₆)alkylsulphonyi, heterocyclyl or aryl, which, in the case of more than one substituent, may be the same or different, or is optionally di-substituted at two adjacent carbon atoms which are available for substitution, to form an aromatic fused bicyclic system; and X is S, SO or SO₂.

In compounds of formula (I) wherein R¹ is formamido, the formamido group can exist in conformations wherein the hydrogen atoms of the -NH-CHO moiety are cis- or trans-; of these the cis conformation normally predominates.

As used herein, the terms thiolactone and lactam refer to lactone ring systems in which the ring oxygen atom is replaced by a sulphur atom and a nitrogen moiety respectively. These may alternatively be referred to as cyclic thioesters and cyclic amides. The nitrogen moiety of a lactam includes a nitrogen atom substituted by hydrogen, C₁₋₆ alkyl, C₁₋₆ alkoxy or aryl.

The term γ-thiolactone or γ-lactam refers to a 5-membered ring bonded via the 3-, 4- or 5- position carbon atom, optionally substituted at the ring carbon atoms as hereinbefore defined, and includes 2-oxodihydro- and 2-oxotetrahydro-thiophene, 2-oxopyrroline and 2-oxopyrrolidine rings. The term δ-thiolactone or δ-lactam refers to a 6-membered ring bonded via the 3-, 4-, 5- or 6-position carbon atom, optionally substituted at the ring carbon atoms as hereinbefore defined, and includes 2-oxodihydro- and 2-oxotetrahydro-thiopyran, 2-oxodihydropyridine and 2-oxopiperidine rings. It will of course be appreciated that a C-4 linked 5-membered ring, since there are no adjacent carbon atoms available for substitution, cannot form an aromatic fused bicyclic system.

When L is other than -CH=, (i.e. the ring R⁴ has no exocyclic double bond) the bonding carbon atom of R⁴ which links the ring to the cephalosporin nucleus may be asymmetric. The present invention includes either stereoisomer as well as mixtures of both isomers. It will be appreciated that only when there is no exocyclic double bond can a six-membered R⁴ ring contain two endocyclic double bonds, in which event the above-mentioned bonding carbon atom of R⁴, which links the ring to the cephalosporin nucleus, cannot be asymmetric.

Since the β-lactam antibiotic compounds of the present invention are intended for use as therapeutic agents in pharmaceutical compositions, it will be readily appreciated that preferred compounds within formula (I) are pharmaceutically acceptable, i.e. are compounds of formula (Ia) or pharmaceutically acceptable salts or pharmaceutically acceptable in vivo hydrolysable esters thereof: 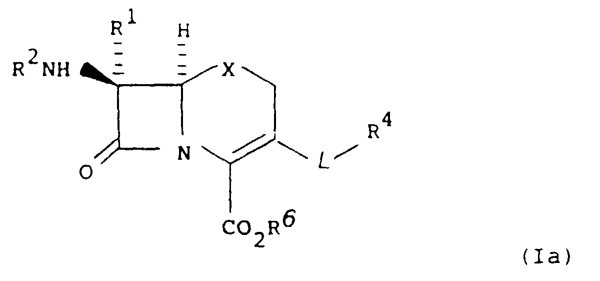 wherein R¹, R², R⁴, X, and L are as defined with respect to formula (I) and the group CO₂R⁶ is CO₂R³ where CO₂R³ is a carboxy group or a carboxylate anion.

Accordingly, the present invention provides a compound of formula (Ia) or a pharmaceutically acceptable salt or in vivo hydrolysable ester thereof, for use as a therapeutic agent, and in particular an in vivo hydrolysable ester thereof for use as an orally administrable therapeutic agent.

The present invention further provides a compound of formula (Ia) or a pharmaceutically acceptable salt or in vivo hydrolysable ester thereof, for use in the treatment of bacterial infections, more particularly an in vivo hydrolysable ester thereof for use in the oral treatment of bacterial infections.

The present invention also includes a method of treating bacterial infections in humans and animals which comprises the administration of a therapeutically effective amount of an antibiotic compound of this invention of the formula (Ia) or a pharmaceutically acceptable in vivo hydrolysable ester thereof, in particular the oral administration of a therapeutically effective amount of an in vivo hydrolysable ester.

In addition, the present invention includes the use of a compound of formula (Ia) or a pharmaceutically acceptable salt or in vivo hydrolysable ester thereof, for the manufacture of a medicament for the treatment of bacterial infections, in particular the use of an in vivo hydrolysable ester for the manufacture of a medicament for the oral treatment of bacterial infections.

Those compounds of the formula (I) wherein R³ is a readily removable carboxy protecting group other than a pharmaceutically acceptable in vivo hydrolysable ester or which are in non-pharmaceutically acceptable salt form are primarily useful as intermediates in the preparation of compounds of the formula (Ia) or a pharmaceutically acceptable salt or pharmaceutically acceptable in vivo hydrolysable ester thereof.

Suitable readily removable carboxy protecting groups for the group R³ include groups forming ester derivatives of the carboxylic acid, including in vivo hydrolysable esters. The derivative is preferably one which may readily be cleaved in vivo.

It will be appreciated that also included within the scope of the invention are salts and carboxy-protected derivatives, including in vivo hydrolysable esters, of any carboxy groups that may be present as optional substituents in compounds of formula (I) or (Ia). Also included within the scope of the invention are acid addition salts of any amino group or substituted amino group that may be present as optional substituents in compounds of formula (I) or (Ia).

Suitable ester-forming carboxyl-protecting groups are those which may be removed under conventional conditions. Such groups for R³ include benzyl, p-methoxybenzyl, benzoylmethyl, p-nitrobenzyl, 4-pyridylmethyl, 5 2,2,2-trichloroethyl, 2,2,2-tribromoethyl, t-butyl, t-amyl, allyl, diphenylmethyl, triphenylmethyl, adamantyl, 2-benzyloxyphenyl, 4-methylthiophenyl, tetrahydrofuran-2-yl, tetrahydropyran-2-yl, pentachlorophenyl, acetonyl, p-toluenesulphonylethyl, methoxymethyl, a silyl, stannyl or phosphorus- containing group, an oxime radical of formula -N=CHR⁷ where R⁷ is aryl or heterocyclic, or an in vivo hydrolysable ester radical such as defined below.

When used herein the term 'aryl' includes phenyl and naphthyl, each optionally substituted with up to five, preferably up to three, groups selected from halogen, mercapto, C₁₋₆alkyl, phenyl, C₁₋₆alkoxy, hydroxy(C₁₋₆)alkyl, C₁₋₆alkylthio, mercapto(C₁₋₆)alkyl, halo(C₁₋₆)alkyl, hydroxy, amino, nitro, carboxy, C₁₋₆alkylcarbonyloxy, alkoxycarbonyl, formyl, or C₁₋₆alkylcarbonyl groups.

The terms 'heterocyclyl' and 'heterocyclic' as used herein include aromatic and non-aromatic, single and fused rings suitably containing up to four hetero-atoms in each ring selected from oxygen, nitrogen and sulphur, which rings may be unsubstituted or substituted by, for example, up to three groups selected from halogen, C₁₋₆alkyl, C₁₋₆alkoxy, halo(C₁₋₆)alkyl, hydroxy, carboxy, carboxy salts, carboxy esters such as C₁₋₆alkoxycarbonyl, C₁₋₆alkoxycarbonyl(C₁₋₆)alkyl, aryl, and oxo groups. Each heterocyclic ring suitably has from 4 to 7, preferably 5 or 6, ring atoms. A fused heterocyclic ring system may include carbocyclic rings and need include only one heterocyclic ring. Compounds within the invention containing a heterocyclyl group may occur in two or more tautometric forms depending on the nature of the heterocyclyl group; all such tautomeric forms are included within the scope of the invention.

When used herein with respect to variable R⁴, the term 'aromatic fused bicyclic system' falls within the definition of 'fused heterocyclic ring system' as described above, wherein at least one ring is a 5- or 6-membered lactone ring. Preferably the second ring is a 5- or 6-membered carbocyclic ring.

When used herein the terms 'alkyl' and 'alkoxy' (or 'lower alkyl' and 'lower alkoxy'), alkenyl and alkynyl include straight and branched chain alkyl groups containing from 1 to 6 carbon atoms, such as methyl, ethyl, propyl and butyl. A particular alkyl group is methyl.

When used herein the term 'halogen' refers to fluorine, chlorine, bromine and iodine.

A carboxyl group may be regenerated from any of the above esters by usual methods appropriate to the particular R³ group, for example, acid- and base- catalysed hydrolysis, or by enzymically-catalysed hydrolysis, or by hydrogenolysis under conditions wherein the remainder of the molecule is substantially unaffected.

Examples of suitable pharmaceutically acceptable in vivo hydrolysable ester groups include those which break down readily in the human body to leave the parent acid or its salt. Suitable ester groups of this type include those of part formulae (i), (ii), (iii), (iv) and (v):

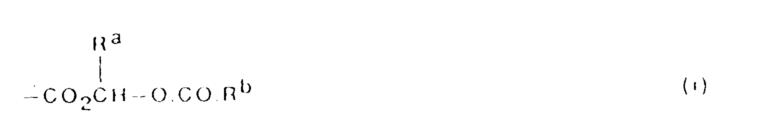

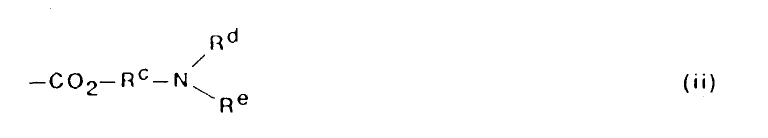

-CO₂CH₂-OR^{f} (iii)

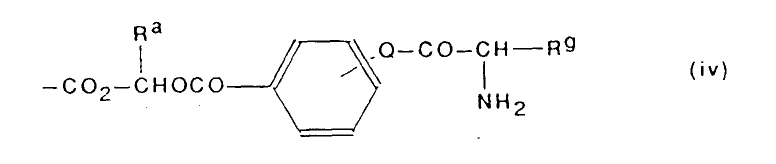

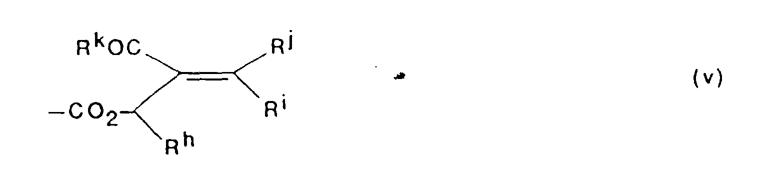

wherein R^{a} is hydrogen, C₁₋₆alkyl, C₃₋₇cycloalkyl, methyl, or phenyl, R^{b} is C₁₋₆alkyl, C₁₋₆alkoxy, phenyl, benzyl, C₃₋₇cycloalkyl, C₁₋₆alkyl(C₃-₇)cycloalkyl, 1-amino(C₁₋₆)alkyl, or 1-(C₁₋₆ alkyl)amino(C₁₋₆)alkyl; or R^{a} and R^{b} together form a 1,2-phenylene group optionally substituted by one or two methoxy groups; R^{c} represents C₁₋₆alkylene optionally substituted with a methyl or ethyl group and R^{d} and R^{e} independently represent C₁₋₆alkyl; R^{f} represents C₁₋₆alkyl; R^{g} represents hydrogen or phenyl optionally substituted by up to three groups selected from halogen, C₁₋₆alkyl, or C₁₋₆alkoxy; and Q is oxygen or NH; R^{h} is hydrogen or _{C1-6}alkyl; Rⁱ is hydrogen, C₁₋₆alkyl optionally substituted by halogen, C₂₋₆alkenyl, C₁₋₆alkoxycarbonyl, aryl or heteroaryl; or R^{h} and Rⁱ together form C₁₋₆alkylene; R^{j} represents hydrogen, C₁₋₆alkyl or C₁₋₆alkoxycarbonyl; and R^{k} represents C₁-₈ alkyl, C₁-₈alkoxy, C₁₋₆alkoxy(C₁₋₆)alkoxy or aryl.

Examples of suitable in vivo hydrolysable ester groups include, for example, acyloxyalkyl groups such as acetoxymethyl, pivaloyloxymethyl, α-acetoxyethyl, α-pivaloyloxyethyl, 1-(cyclohexylcarbonyloxy)prop-1-yl, and (1-aminoethyl)carbonyloxymethyl; alkoxycarbonyloxyalkyl groups, such as ethoxycarbonyloxymethyl, α-ethoxycarbonyloxyethyl and propoxycarbonyloxyethyl; dialkylaminoalkyl especially di-loweralkylamino alkyl groups such as dimethylaminomethyl, dimethylaminoethyl, diethylaminomethyl or diethylaminoethyl; 2-(alkoxycarbonyl)-2-alkenyl groups such as 2-(isobutoxycarbonyl)pent-2-enyl and 2-(ethoxycarbonyl)but-2-enyl; lactone groups such as phthalidyl and dimethoxyphthalidyl; and esters linked to a second β-lactam antibiotic or to a β-lactamase inhibitor.

A preferred in vivo hydrolysable ester group is the pivaloyloxymethyl ester.

A further suitable pharmaceutically acceptable in vivo hydrolysable ester group is that of the formula: 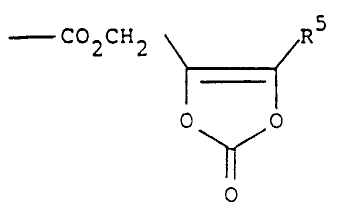 wherein R⁵ is hydrogen, C₁₋₆ alkyl or phenyl.

Suitable pharmaceutically acceptable salts of the carboxy group of the compound of formula (I) include metal salts, eg aluminium, alkali metal salts such as sodium or potassium especially sodium, alkaline earth metal salts such as calcium or magnesium, and ammonium or substituted ammonium salts, for example those with lower alkylamines such as triethylamine, hydroxy-lower alkylamines such as 2-hydroxyethylamine, bis-(2-hydroxyethyl)amine or tris-(2-hydroxyethyl)- amine, cycloalkylamines such as dicyclohexylamine, or with procaine, dibenzylamine, N,N-dibenzylethylene- diamine, 1-ephenamine, N-methylmorpholine, N-ethylpiperidine, N-benzyl-β-phenethylamine, dehydroabietylamine, N,N'-bisdehydro-abietylamine, ethylenediamine, or bases of the pyridine type such as pyridine, collidine or quinoline, or other amines which have been used to form salts with known penicillins and cephalosporins. Other useful salts include the lithium salt and silver salt. Salts within compounds of formula (I), may be prepared by salt exchange in conventional manner.

In compounds of formula (I) or (Ia), the group X may be sulphur or an oxidised sulphur atom, i.e. a sulphoxide (SO) or sulphone (SO₂) group. When X is a sulphoxide group it will be understood that α- and β-isomers may exist; both such isomers are encompassed within the scope of the present invention.

Preferably, X is sulphur.

Advantageously, R¹ is hydrogen.

Suitable acyl groups R² include those of formulae (a) - (f):

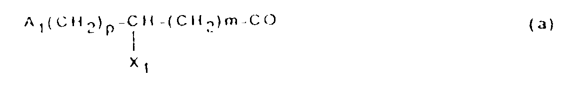

A₂CO- (b)

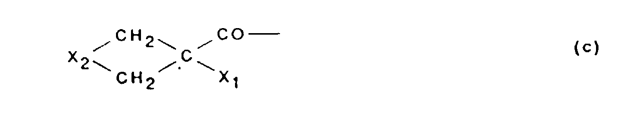

A₂-X₃-(CH₂)ₚ-CO- (d)

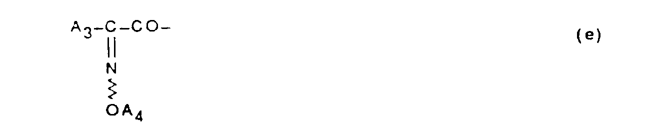

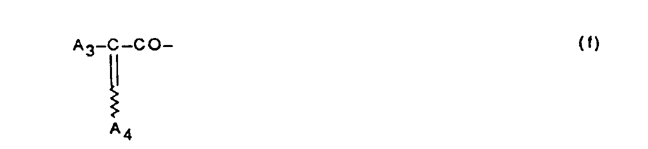

wherein p is 0, 1 or 2; m is 0, 1 or 2; A₁ is C₁₋₆ alkyl, substituted C₁₋₆alkyl, C₃₋₆cycloalkyl, cyclohexenyl, cyclohexadienyl, an aromatic (including heteroaromatic) group, such as phenyl, substituted phenyl, thienyl, pyridyl, or an optionally substituted thiazolyl group, a C₁₋₆alkylthio or C₁₋₆alkyloxy group; X₁ is a hydrogen or halogen atom, a carboxylic acid, carboxylic ester, sulphonic acid, azido, tetrazolyl, hydroxy, acyloxy, amino, ureido, acylamino, heterocyclylamino, guanidino or acylureido group; A₂ is an aromatic group, for example a phenyl, 2,6-dimethoxyphenyl,2-alkoxy-1-naphthyl, 3-arylisoxazolyl, or a 3-aryl-5-methylisoxazolyl group, such as 3-(2-chloro-6-fluorophenyl)-5-methylisoxazol- 4-yl; a substituted alkyl group; or a substituted dithietane; X₂ is a -CH₂OCH₂-, -CH₂SCH₂- or alkylene group; X₃ is an oxygen or sulphur atom; A₃ is an aryl or heteroaryl group such as phenyl, substituted phenyl, furyl, aminothiazolyl or aminothiadiazolyl in which the amino group is optionally protected; and A₄ is hydrogen, C₁₋₆alkyl, C₃-₈cycloalkyl, C₃-₈cycloalkyl(C₁₋₆)alkyl, C₁₋₆alkoxycarbonyl(C₁₋₆) alkyl, C₂-₆alkenyl, carboxy(C₁₋₆)alkyl, C₂-₆alkynyl, aryl, or C₁₋₆alkyl substituted by up to three aryl groups.

The term 'heteroaryl' as used herein means a heteroaromatic heterocyclic ring or ring system, suitably having 5 or 6 ring atoms in each ring.

Suitably when R² is a group (a), A₁ is C₁₋₆alkyl, C₃-₆cycloalkyl, cyclohexenyl, cyclohexadienyl, phenyl, substituted phenyl such as hydroxyphenyl, thienyl or pyridyl; and X₁ is a hydrogen or halogen atom, or a carboxy, carboxylic ester, azido, tetrazolyl, hydroxy, acyloxy, optionally protected amino, ureido, guanidino or acylureido group. In an example of R² when a group of formula (a), A₁ is phenyl, X₁ is hydrogen and p and m are 0.

Suitably when R² is a group of formula (d), A₂ is phenyl, X₃ is oxygen and p is 1.

Alternatively when R² is a group of formula (e), suitable values for the group A₃ include those commonly found in antibacterially active cephalosporins containing a hydroxyimino or substituted hydroxyimino group in the side chain attached to position 7 of the cephalosporin nucleus, for example phenyl, thien-2-yl, thien-3-yl, fur-2-yl, fur-3-yl, pyrid-2-yl, pyrid-3-yl, pyrid-4-yl, 5-amino-1,2,4-thiadiazol-3-yl and 2-aminothiazol-4-yl in each of which the amino group is optionally protected.

Preferred groups for A₃ include phenyl, 2-aminothiazol-4-yl, furan-2-yl, thien-2-yl, 2-(2-chloroacetamido)thiazol-4-yl, 2-tritylamino- thiazol-4-yl, 5-amino-1,2,4-thiadiazol-3-yl and 4-aminopyrimid-2-yl.

In compounds of formula (Ia), a particularly preferred group for A₃ is 2-aminothiazol-4-yl.

Suitable values for the group A₄ include hydrogen, methyl, ethyl, cyclopropylmethyl, triphenylmethyl (trityl), cyclobutyl, cyclopentyl, cyclohexyl, cycloheptyl, cyclooctyl, phenyl, carboxymethyl, carboxypropyl and t-butoxycarbonylmethyl.

Preferred values for A₄ in compounds of formula (Ia) include methyl and hydrogen.

It will be appreciated that compounds of the invention wherein R² is a group of formula (e) (or (f)) can exist as syn and anti (or E and Z) isomers or mixtures thereof. Both isomers are encompassed within the scope of this invention.

Preferably the compounds of the invention wherein R² is a group of formula (e) have the syn configuration (i.e. have the group OA₄ syn to the amide linkage) or are enriched in that isomer.

Similarly, when R² is a group of formula (f), the group A₄ is preferably cis to the amide linkage, i.e. when group (f) is 2-amino-thiazol-4-yl, the Z-configuration is preferred.

Examples of R⁴ groups include 2,5-dihydro-2-oxothienyl, 2-oxotetrahydrothienyl, and 2-oxopyrrolidinyl derivatives, for example N-methoxy- and N-methyl-2-oxopyrrolidinyl derivatives.

Examples of L include (CH₂)ₙ where n = 0, and -(CH₂)ₓ-Y-(CH₂)_{y} where Y is sulphur, x is 1 and y is 0, and -(CH₂)ₓ-Y-(CH₂)_{y}- where Y is sulphur and x and y are 0.

Certain compounds of the invention include an amino group which may be protected. Suitable amino protecting groups are those well known in the art which may be removed under conventional conditions without disruption of the remainder of the molecule.

Examples of amino protecting groups include C₁₋₆ alkanoyl; benzoyl; benzyl optionally substituted in the phenyl ring by one or two substituents selected from C₁₋₄alkyl, C₁₋₄alkoxy, trifluoromethyl, halogen, or nitro; C₁₋₄alkoxycarbonyl; benzyloxycarbonyl or trityl substituted as for benzyl above; allyloxycarbonyl,trichloroethoxycarbonyl or chloroacetyl.

Some of the compounds of this invention may be crystallised or recrystallised from solvents such as organic solvents. In such cases solvates may be formed. This invention includes within its scope stoichiometric solvates including hydrates as well as compounds containing variable amounts of water that may be produced by processes such as lyophilisation.

Since the antibiotic compounds of the invention are intended for use in pharmaceutical compositions it will readily be understood that they are each provided in substantially pure form, for example at least 60% pure, more suitably at least 75% pure and preferably at least 85%, especially at least 95% pure (% are on a weight for weight basis). Impure preparations of the compounds may be used for preparing the more pure forms used in the pharmaceutical compositions; these less pure preparations of the compounds should contain at least 1%, more suitably at least 5% and preferably from 10 to 49% of a compound of the formula (I) or salt thereof.

Specific compounds within this invention of formula (Ia) include the following pharmaceutically acceptable carboxylic acids salts and in-vivo hydrolysable esters:
sodium (6R,7R)-7-[2-(2-aminothiazol-4-yl)-2-(Z)-methoxyimino-acetamido]-3-[(2,5-dihydro-2-oxothien-4-yl)thiomethyl]ceph-3-em-4-carboxylate;
pivaloyloxymethyl (6R,7R)-7-[2-(2-aminothiazol-4-yl)-2-(Z)-methoxyiminoacetamido]-3-[2,5-dihydro-2-oxothien-4-yl)thiomethyl]ceph-3-em-4-carboxylate;
sodium (6R,7R)-7-[2-(2-aminothiazol-4-yl)-2-(Z)-methoxyiminoacetamido]-3-[(N-methoxy-2-oxopyrollidin-3-yl)thio]ceph-3-em-4-carboxylate;
pivaloyloxymethyl (6R,7R)-7-[2-(2-aminothiazol-4-yl)-2-(Z)-methoxyiminoacetamido]-3-[N-methoxy-2-oxopyrrollidin-3-yl)thio]ceph-3-em-4-carboxylate;
sodium (6R,7R)-7-[2-(2-aminothiazol-4-yl)-2-(Z)-methoxyiminoacetamido]-3-[(N-methyl-2-oxopyrrolidin-3-yl)thio]ceph-3-em-4-carboxylate;
sodium (6R, 7R)-7-[2-(2-aminothiazol-4-yl)-2-(Z)-methoxyiminoacetamido]-3-[(2-oxotetrahydrothien-3-yl)thio]ceph-3-em-4-carboxylate;
pivaloyloxymethyl (6R,7R)-7-[2-(2-aminothiazol-4-yl)-2-(Z)-methoxyiminoacetamido]-3-[(2-oxotetrahydrothien-3-yl)thio]ceph-3-em-4-carboxylate;
sodium (6R,7R)-7-[2-(2-aminothiazol-4-yl)-2-(Z)-methoxyiminoacetamido]-3-(2-oxotetrahydrothien-5-yl)ceph-3-em-4-carboxylate;
sodium (6R,7R)-7-[2-(2-aminothiazol-4-yl)-2-(Z)-methoxyiminoacetamido]-3-[(2, 5-dihydro-2-oxothien-4-yl)thio]ceph-3-em-4-carboxylate; and
sodium (6R,7R)-7-[2-(2-aminothiazol-4-yl)-2-(Z)-pentenamido-3-[(2-oxotetrahydrothien-3-yl)thio]ceph-3-em-4-carboxylate.

The present invention further provides a process for the preparation of a compound of formula (I), which process comprises treating a compound of formula (II) or a salt thereof: 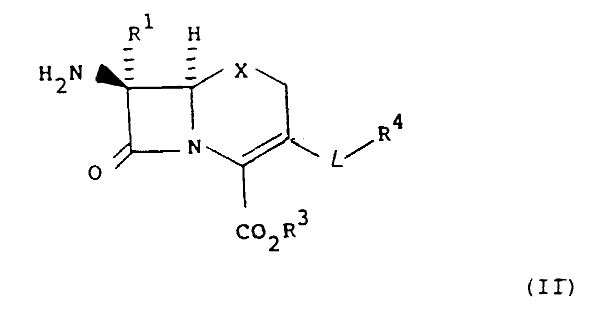 wherein R¹, R³, R⁴, X and L are as hereinbefore defined, wherein any reactive groups may be protected, and wherein the amino group is optionally substituted with a group which permits acylation to take place; with an N-acylating derivative of an acid of formula (III):

R²OH (III)

wherein R² is as defined with respect to formula (I) and wherein any reactive groups may be protected; and thereafter, if necessary or desired, carrying out one or more of the following steps:
i) removing any protecting groups;
ii) converting the group CO₂R³ into a different group CO₂R³;
iii) converting the group R² into a different group R²;
iv) converting the group X into a different group X;
v) reducing any endocyclic double bond within R⁴;
5 vi) reducing an optional exocyclic double bond adjacent to R⁴;
vii) converting the product into a salt.

Acids of formula (III) may be prepared by methods known in the art, or methods analogous to such processes. Suitable processes include those described, for example, in UK Patent 2 107 307 B, UK Patent Specification No. 1,536,281, and U.K. Patent Specification No. 1,508,064.

Suitable groups which permit acylation to take place and which are optionally present on the amino group of the starting material of the formula (II) include N-silyl, N-stannyl and N-phosphorus groups, for example trialkylsilyl groups such as trimethylsilyl, trialkyltin groups such as tri-n-butyltin, groups of formula -P.R²⁰R²¹ wherein R²⁰ is an alkyl, haloalkyl, aryl, aralkyl, alkoxy, haloalkoxy, aryloxy, aralkyloxy or dialkylamino group, R²¹ is the same as R²⁰ or is halogen or R²⁰ and R²¹ together form a ring; suitable such phosphorus groups being -P(OC₂H₅)₂, -P(C₂H₅)₂, 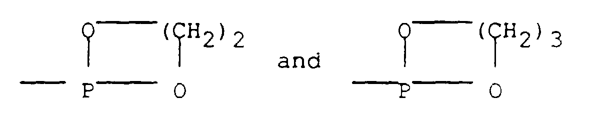 A group which may optionally be introduced onto the amino group in the compound of formula (II) is trimethylsilyl.

Advantageously the silylation reaction may be carried out in situ, prior to the acylation reaction, with a silylating agent that does not require concomitant addition of base. Suitable silylating agents include, for example, N-(trimethylsilyl)-acetamide, N,O-bis-(trimethylsilyl)acetamide, N,O-bis(trimethylsilyl)-trifluoroacetamide, N-methyl-N-trimethylsilylacetamide, N-methyl-N-trimethylsilyl-trifluoroacetamide, N,N'-bis(trimethylsilyl)urea, and N,O-bis(trimethylsilyl)carbamate. A preferred silylating agent is N,O-bis(trimethylsilyl)acetamide. The silylation reaction may suitably be carried out in an inert, anhydrous organic solvent such as dichloromethane at room temperature or at an elevated temperature, for example 30 - 60°C, preferably 40 - 50°C.

The above process may optionally be carried out in the presence of a small quantity, for example 0.1 equivalents, of a silyl halide, for example a tri(C₁₋₆)alkylslkyl halide, especially trimethylsilyl chloride.

A reactive N-acylating derivative of the acid (III) is employed in the above process. The choice of reactive derivative will of course be influenced by the chemical nature of the substituents of the acid.

Suitable N-acylating derivatives include an acid halide, preferably the acid chloride or bromide, or alternatively a symmetrical or mixed anhydride. The acylation may be effected in the presence of an acid binding agent for example, a tertiary amine, molecular sieves, an inorganic base (such as calcium carbonate or sodium bicarbonate) or when an acid halide is used, an oxirane which binds hydrogen halide liberated in the acylation reaction. The oxirane is preferably a (C₁₋₆)-1,2-alkylene oxide such as ethylene oxide or propylene oxide. The acylation reaction may be carried out at a temperature in the range -50°C to +50°C, preferably -20°C to +20°c, in aqueous or non-aqueous media such as water, acetone, tetrahydrofuran, ethyl acetate, dimethylacetamide, dimethylformamide, acetonitrile, dichloromethane, 1,2-dichloroethane, or mixtures thereof.

Alternatively, the reaction may be carried out in an unstable emulsion of water-immiscible solvent, especially an aliphatic ester or ketone, such as methyl isobutyl ketone or butyl acetate. The acylation with acid halide or anhydride is suitably carried out in the presence of a basic catalyst such as pyridine or 2,6-lutidine.

Acid halides may be prepared by reacting the acid (III) or a salt or a reactive derivative thereof with a halogenating (eg chlorinating or brominating) agent such as phosphorus pentachloride, thionyl chloride, oxalyl chloride or phosgene.

Suitable mixed anhydrides are anhydrides with, for example, carbonic acid monoesters, trimethyl acetic acid, thioacetic acid, diphenylacetic acid, benzoic acid, phosphorus acids (such as phosphoric, phosphorous, and phosphinic acids) or aromatic or aliphatic sulphonic acids (such as p-toluenesulphonic acid or methanesulphonic acid).

Alternative N-acylating derivatives of acid (III) are the acid azide, or activated esters such as esters with 2-mercaptopyridine, cyanomethanol, p-nitrophenol, 2,4-dinitrophenol, thiophenol, halophenols, including pentachlorophenol, monomethoxyphenol, N-hydroxy succinimide, N-hydroxybenzotriazole, or 8-hydroxyquinoline; or amides such as N-acylsaccharins, N-acylthiazolidin-2-thione or N-acylphthalimides; or an alkylidene iminoester prepared by reaction of the acid (III) with an oxime.

Other reactive N-acylating derivatives of the acid (III) include the reactive intermediates formed by reaction in situ with a condensing agent such as a carbodiimide, for example, N,N'-diethyl-, dipropyl- or diisopropylcarbodiimide, N,N'-di-cyclohexyl-carbodiimide, or N-ethyl-N'-(3-(dimethylamino)propyl]carbodiimide; a suitable carbonyl compound, for example, N,N'-carbonyldiimidazole or N,N'-carbonylditriazole;
an isoxazolinium salt, for example, N-ethyl-5-phenylisoxazolinium-3-sulphonate or N-t-butyl-5-methylisoxazolinium perchlorate; or an N-alkoxycarbonyl-2-alkoxy1,2-dihydroquinoline, such as N-ethoxycarbonyl-2-ethoxy-1,2-dihydroquinoline. Other condensing agents include Lewis acids (for example BBr₃ - C₆H₆);
or a phosphoric acid condensing agent such as diethylphosphorylcyanide. The condensation reaction is preferably carried out in an organic reaction medium, for example, methylene chloride, dimethylformamide, acetonitrile, alcohol, benzene, dioxan or tetrahydrofuran.

A further method of forming the N-acylating derivative of the acid of formula (III) is to treat the acid of formula (III) with a solution or suspension preformed by addition of a carbonyl halide, preferably oxalyl chloride, or a phosphoryl halide such as phosphorus oxychloride, to a halogenated hydrocarbon solvent, preferably dichloromethane, containing a lower acyl tertiary amide, preferably N,N-dimethylformamide. The N-acylating derivative of the acid of formula (III) so derived may then be caused to react with a compound of formula (II). The acylation reaction may conveniently be carried out at -40° to +30°C, if desired in the presence of an acid binding agent such as pyridine. A catalyst such as 4-dimethylaminopyridine may optionally also be added. A preferred solvent for the above acylation reaction is dichloromethane.

The optional reduction steps, the optional conversion of R² to a different R², CO₂R³ to a different CO₂R³ and X to a different X, and the optional formation of a salt, may be carried out using methods well known in the art of cephalosporin and penicillin chemistry.

For example, when the group X is S, SO, or SO₂, the group X may be converted into a different group X by methods of oxidation or reduction well known in the art of cephalosporin and penicillin synthesis, as described, for example, in European Patent Application Publication No. 0 114 752. For example, sulphoxides (in which X is SO) may be prepared from the corresponding sulphide (in which X is S) by oxidation with a suitable oxidising agent, for example an organic peracid such as m-chloroperbenzoic acid.

Reduction steps, for example reduction of any endocyclic double bond within R⁴ and/or reduction of an exocyclic double bond adjacent to R⁴, are generally effected by process of catalytic hydrogenation in the presence of a suitable catalyst or combination thereof.

In the process described hereinabove, and in the processes described hereinbelow, it may be necessary to remove protecting groups. Deprotection may be carried out by any convenient method known in the art such that unwanted side reactions are minimised. Separation of unwanted by-products may be carried out using standard methods.

Compounds of formula (II) are novel compounds and as such form part of the invention. Compounds of formula (II) may be prepared by removal of R² from compounds of formula (I) prepared by the process described hereinbelow.

In a further process of the invention, compounds of formula (I) may be prepared according to one of process variants (a) to (c):
(a) by treating a compound of formula (IV): 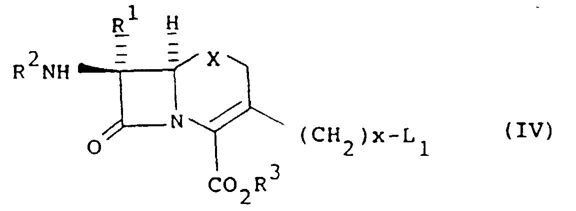 wherein X, R¹, R², x and R³ are as hereinbefore defined with respect to formula (I), and L₁ is a Y-group precursor (or a leaving group), with a compound of formula (V):

   L₂-(CH₂)_{y}-R⁴ (V)

   wherein L₂ is a leaving group (or a Y-group precursor) and R⁴ and y are as hereinbefore defined with respect to formula (I) (to give a compound of formula (I) in which L is -(CH₂)ₓ-Y-(CH₂)_{y}-);
(b) cyclising a compound of formula (VI): 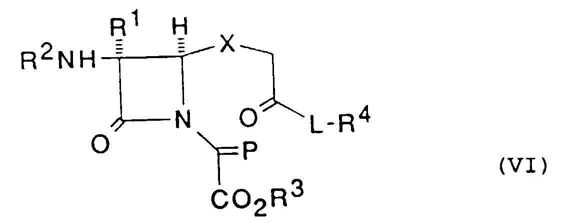 wherein X, R¹, R², R³ and R⁴ are as hereinbefore defined with respect to formula (I), L is -(CH₂)ₙ as hereinbefore defined, and P' is a phosphorus residue (to give a compound of formula (I) in which L is -(CH₂)ₙ-;
(c) treating a compound of formula (VII): 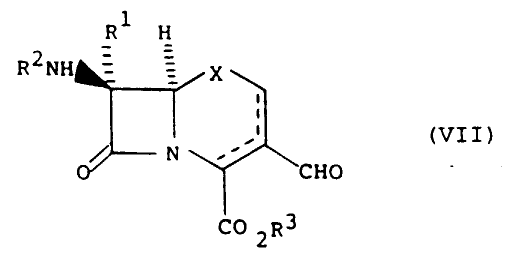 wherein X, R¹, R² and R³ are as hereinbefore defined with respect to formula (I) and the dashed line represents a double bond in the 2- or 3-position of the cephalosporin nucleus, with a phosphorus ylid compound of formula (VIII):

   P"=R⁴ (VIII)

   wherein P'' is the phosphorus residue and R⁴ is as hereinbefore defined with respect to formula (I) (to give a compound of formula (I) in which L is -CH=);
and thereafter as necessary or desired, carrying out one or more of the following steps:
i) removing any protecting groups;
ii) converting the group CO₂R³ into a different group CO₂R³;
iii) converting the group R² into a different group R²;
iv) converting the group X into a different group X;
v) reducing any endocyclic double bond within R⁴;
vi) reducing an exocyclic double bond adjacent to R⁴ (L = -CH=);
vii) converting the product into a salt.

In process variant (a), where L₁ in the compound of formula (IV) is a Y-group precursor, suitably the corresponding thiol group, L₂ in the compound of formula (V) may be a leaving group, for example a halogen leaving group such as bromo. The reaction may be carried out under standard conditions for nucleophilic substitution reactions, typically in the presence of a base in an inert solvent at room temperature or with heating, as dictated by the lability of the leaving group, L₂.

Alternatively, where x is 0 and Y is oxygen, L₂ may be a hydroxyl group and the reaction may be carried out under 'Mitsunobu' conditions in the presence of triphenylphosphine and a dialkyl azodicarboxylate, for example diethyl azodicarboxylate.

Where L₁ is a leaving group, L₂ is suitably a Y-group precursor such as thiol. A typical leaving group L₁ is mesyloxy or a halogen group such as chloro and the reaction may be carried out at ambient temperature in an inert solvent optionally in the presence of a base, for example a tertiary amine.

A reaction in which L₁ is a leaving group and L₂ is a Y-group precursor may also be carried out under 'Mitsunobu' conditions by reacting a compound of formula (IV) in which x is 1 and L₁ is hydroxy with a compound of formula (V) in which y is 0 and L₂ is a hydroxyl group.

Compounds of formula (IV) in which L₁ is a hydroxyl group or a thiol group are known compounds or may be prepared from known compounds. Compounds of formula (IV) in which x is 0 or 1 and L₁ is -OH or -SH may be prepared using procedures described by R. Scartazzini and H. Bickel, Helv. Chim. Acta., 57, 1919-34, (1974); E.M. Gordon and C.M. Cimarusti, Tett. Lett., 16, 1359, (1977) and GB Patent 1 516 655. Alternatively a compound of formula (IV) in which x is 0 and L₁ is the Y-group precursor, -SH, may be prepared from the corresponding compound in which L₁ is a leaving group such as mesyloxy, by reaction with sodium bisulphide, or a leaving group such as methoxy, by reaction with sodium bisulphide in the presence of benzyltrimethyl ammonium chloride and 1,8-diazabicyclo[5.4.0]undec- 7-ene.

Compounds of formula (IV) in which L₁ is a leaving group, for example mesyloxy or halogen may be prepared by standard methodology from the corresponding compound in which L₁ is hydroxy. Certain compounds of formula (IV) are commercially available.

Compounds of formula (V) are either known compounds or may be prepared from known starting materials by standard methods in the art of lactone chemistry.

In process variant (b), the cyclisation reaction is an intramolecular Wittig-type reaction and is typically carried out by heating the compound of formula (VI) in an organic solvent system, for example in toluene, optionally in the presence of a suitable acid such as benzoic acid.

The phosphorus residue, P' is typically a trialkylphosphoranylidene residue, for example a C₁₋₆ trialkylphosphoranylidene residue such as tri-n-butylphosphoranylidene, or a triarylphosphoranylidene residue such as triphenylphosphoranylidene.

A compound of formula (VI) may be prepared from a compound of formula (IX): 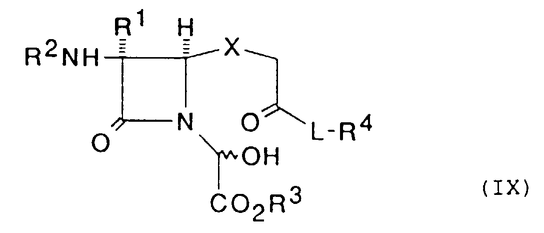 wherein X, R¹, R², R³, L and R⁴ are as hereinbefore defined, by reaction with a halogenating agent, suitably a chlorinating agent such as thionyl chloride, which reaction displaces the formula (IX) hydroxyl group by halogen, suitably chloride, and is typically carried out at reduced temperature in an inert solvent, for example in tetrahydrofuran, in the presence of a base, typically a pyridine derivative such as 2,6-lutidine. Formation of the phosphorane may be effected by treatment of the 5 halo-intermediate with an appropriate phosphine derivative, for example tri-n-butylphosphine or triphenylphosphine, suitably at ambient temperature in an inert solvent such as dioxan.

A compound of formula (IX) may be prepared by reaction of the corresponding azetidin-2-one compound of formula (X): 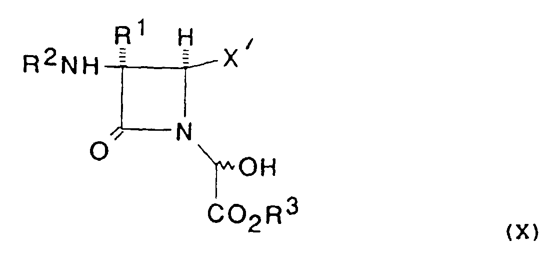 wherein R¹, R² and R³ are as hereinbefore defined and X' is an X-group precursor (or a leaving group), with a compound of formula (XI):

L₃-CH₂-C(O)-L-R⁴ (XI)

wherein R⁴ and L are as hereinbefore defined and L₃ is a leaving group (or an X-group precursor).

In a typical preparation of a compound of formula (IX) in which X is sulphur, an L₃ leaving group in a compound of formula (XI), suitably a halogen group such as chloro or bromo, is displaced by an X' mercapto group in a compound of formula (X). The reaction may be carried out at ambient temperature in an inert solvent, for example acetone, with the addition of a base, for example potassium carbonate, before work-up.

Azetidin-2-one compounds of formula (X) may be prepared according to known methods in heterocyclic synthetic chemistry and particularly by known methods in the art of β-lactam chemistry.

For example, a compound of formula (X) in which X' is a mercapto group may be prepared by ring opening of a 4-thia-2,6-diazabicyclo[3.2.0]hept-2-ene-7-one derivative according to the method of Masayuki Narisada et al., Tetrahedron Lett., 1755, (1978).

Compounds of formula (XI) are known compounds or may be prepared by standard methodology. For example, the compounds of formula (XI) in which L₃ is chloro or bromo may be prepared from the corresponding carboxylic acid (L₃=COOH) via formation of the acid chloride followed by treatment with diazomethane and reaction of the resulting diazo compound with hydrogen chloride or hydrogen bromide.

In process variant (c), the reaction between a compound of formula (VII) and a phosphorus ylid of formula (VIII) is a Wittig-type reaction and is typically carried out at ambient temperature in an inert solvent such as dichloromethane.

The phosphorus ylid of formula (VIII) is typically a phosphonium ylid (or phosphorane). Suitable phosphonium ylids include compounds of formula (VIII) in which P'' is a trialkylphosphoranylidene residue, for example a C₁₋₆ trialkylphosphoranylidene residue such as tri-n-butylphosphoranylidene or a triarylphosphoranylidene residue such as triphenylphosphoranylidene.

Phosphonate reagents may also be used. These include compounds containing a dialkylphosphonate residue, for example a di-C₁₋₆ alkylphosphonate residue such as diethylphosphonate.

A phosphonium ylid may be prepared in situ by the action of base, for example sodium hydride, on the corresponding phosphonium halide, for example the corresponding phosphonium bromide, in dimethylsulphoxide.

Compounds of formula (VIII) may be prepared by methods well known in the art of phosphorus chemistry.

It should be noted that in processes of this invention Δ²-cephems, for example compounds of formula (IV), may function as intermediates, in the synthetic sequences. Subsequent isomerisation steps by methods well known in cephalosporin chemistry will provide the Δ³-cephems of the invention.

The present invention also provides a pharmaceutical composition which comprises a compound of formula (Ia) or a pharmaceutically acceptable salt or in vivo hydrolysable ester thereof and a pharmaceutically acceptable carrier. The compositions of the invention include those in a form adapted for oral, topical or parenteral use and may be used for the treatment of bacterial infection in mammals including humans.

The antibiotic compounds according to the invention may be formulated for administration in any convenient way for use in human or veterinary medicine, by analogy with other antibiotics.

The composition may be formulated for administration by any route, such as oral, topical or parenteral, especially oral. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles which may include edible oils for example almond oil, oily esters for example esters of glycerine, propylene glycol, glycerine, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing. Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 to 3000 mg per day, for instance 1500 mg per day depending on the route and frequency of administration.

Such a dosage corresponds to 1.5 to 50 mg/kg per day. Suitably the dosage is from 5 to 20 mg/kg per day.

No toxicological effects are expected when a compound of formula (Ia) or a pharmaceutically acceptable salt or in vivo hydrolysable ester thereof is administered in the above-mentioned dosage range.

The compound of formula (Ia) may be the sole therapeutic agent in the compositions of the invention or a combination with other antibiotics or with a β-lactamase inhibitor may be employed.

Advantageously, the compositions also comprise a compound of formula (XII) or a pharmaceutically acceptable salt or ester thereof: 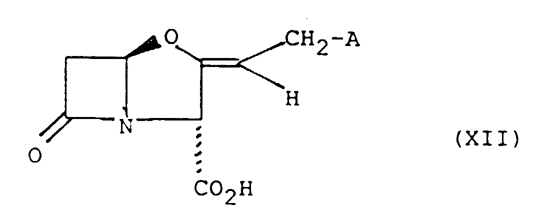 wherein
A is hydroxyl, substituted hydroxyl, thiol, substituted thiol, amino, mono- or di-hydrocarbyl-substituted amino, or mono- or di-acylamino, an optionally substituted triazolyl group; or an optionally substituted tetrazolyl group as described in EP-A-O 053 893.

A further advantageous composition comprises a compound of formula (Ia) or a pharmaceutically acceptable salt or in vivo hydrolysable ester thereof together with a compound of formula (XIII) or a pharmaceutically acceptable salt or in vivo hydrolysable ester thereof: 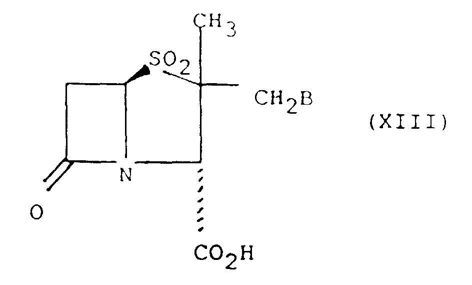 wherein
B represents hydrogen, halogen or a group of formula:
in which R⁸ and R⁹ are the same or different and each represents hydrogen, C₁₋₆ alkoxycarbonyl or carboxy, or a pharmaceutically acceptable salt thereof.

Further suitable β-lactamase inhibitors include 6-alkylidene penems of formula (XIV): 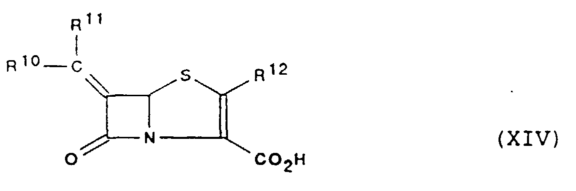 or a pharmaceutically acceptable salt or in vivo hydrolysable ester thereof, wherein R¹⁰ and R¹¹ are the same or different and each represents hydrogen, or a C₁₋₁₀ hydrocarbon or heterocyclic group optionally substituted with a functional group; and R¹² represents hydrogen or a group of formula R¹³ or -SR¹³ where R¹³ is an optionally substituted C₁₋₁₀ hydrocarbon or heterocyclic group, as described in EP-A-0 041 768.

Yet further suitable β-lactamase inhibitors include 6β-bromopenicillanic acid and pharmaceutically acceptable salts and in vivo hydrolysable esters thereof and 6β-iodopenicillanic acid and pharmaceutically acceptable salts and in vivo hydrolysable esters thereof described in, for example, EP-A-0 410 768 and EP-A-0 154 132 (both Beecham Group).

Such compositions of this invention which include a β-lactamase inhibitory amount of a β-lactamase inhibitor are formulated in a conventional manner using techniques and procedures per se known in the art.

The antibiotic compounds of the present invention are active against a wide range of organisms including both Gram-negative organisms such as E.coli and Gram-positive organisms such as S.aureus.

The following Examples illustrate the preparation of compounds of the invention and intermediates thereto. The following biological data illustrate the activity of compounds of the invention in the form of MIC values (minimum inhibitory concentration) against a sample E.coli organism (NCTC 10418) and a sample S.aureus organism (S.aureus Oxford).

### Example 1

### Sodium (6R,7R)-7-[2-(2-Aminothiazol-4-yl)-2-(Z)-methoxyiminoacetamido]-3-[(2,5-dihydro-2-oxothien -4-yl)thiomethyl]ceph-3-em-4-carboxylate

### (a) Diphenylmethyl(6R,7R)-3-[(2,5-Dihydro-2-oxothien-4-yl)thiomethyl]-7-phenylacetamidoceph-3-em-4-carboxylate

Thiotetronic acid (3.0g) was dissolved in dry dioxan (30ml) before treating with phosphorous pentasulphide (1.15g). The heterogeneous mixture was warmed to 80°C and stirred for 2h. Evaporation of the solvent in vacuo gave a residue which was partitioned between chloroform and water. The organic phase was washed with brine and dried over anhydrous magnesium sulphate. Removal of the solvent gave crude (2,5-dihydro-4-mercapto)thiophen-2-one (2.15g). This was immediately dissolved in dry dichloromethane (50ml) and treated with diphenylmethyl (6R,7R)-3-chloromethyl-7-phenylacetamidoceph-3-em-4-carboxylate (8.70g) and N,N-diisopropylethylamine (2.83ml). The reaction was stirred at room temperature for 1h. Evaporation of the solvent gave a residue which was partitioned between ethyl acetate and water. The organic phase was washed with water and brine before drying over anhydrous magnesium sulphate. Chromatography on silica gel (Kieselgel) eluting with 1:1 ethyl acetate:hexane followed by 7:3 ethyl acetate:hexane gave an oil which was crystallised from ethyl acetate to give the title compound (4.89g), m.p. 186-187°C from ethyl acetate (Found: C, 62.88; H, 4.38; N, 4.47; S, 15.15; C₃₃H₂₈N₂O₅S₃ requires; C, 63.04; H, 4.49; N, 4.45; S, 15.30); νₘₐₓ(CH₂Cl₂) 3400, 1790, 1730, and 1680cm⁻¹; δ_{H}(CDCl₃) 3.35 (1H, d, J 18.3Hz), 3.56 (1H, d, J 18.3Hz), 3.59 (1H, d, J 16.2Hz), 3.68 (1H, d, J 16.2Hz), 3.88 (1H, d, J 12.3Hz), 3.95 (2H, s) superimposed on 3.95 (1H, d, J 4.8Hz), 5.84-5.88 (2H, m) 6.03 (1H, d, J 8.9Hz), 6.95 (1H, s), and 7.25-7.39 (15H, m).

### (b) Diphenylmethyl (6R,7R)-7-Amino-3-[(2,5-dihydro-2-oxothien-4-yl)thiomethyl]ceph-3-em-4-carboxylate

Diphenylmethyl (6R,7R)-3-[(2,5-dihydro-2-oxothien-4-yl)thiomethyl]-7-phenylacetamidoceph-3-em-4-carboxylate (837mg) was dissolved in dichloromethane (10ml) under an atmosphere of argon. The reaction was cooled to -20°C and treated with N-methylmorpholine (290µl) and a phosphorus pentachloride solution in dichloromethane (10.37ml of 40mg ml-¹ solution). Stirring was continued at -20°C for 30 min. Methanol (2.7ml) was added and the reaction stirred at room temperature for 30 min before adding water (3.6ml) and stirring for 1h. The solvent was removed by evaporation in vacuo and the residue redissolved in ethyl acetate and water. 1.0M Aqueous ammonia was added to adjust the pH to 6.5 before separating the organic phase, washing with water and brine and finally drying over anhydrous magnesium sulphate. The crude material was chromatographed on silica gel (Kieselgel) eluting with 1:1 ethyl acetate:hexane followed by neat ethyl acetate to give the title compound finally isolated as a foam (275mg), νₘₐₓ(CHCl₂) 1790, 1725, and 1680cm⁻¹; δ_{H}(CDCl₃) 3.40 (1H, d, J 18.2Hz), 3.60 (1H, d, J 18.2Hz), 3.76-3.95 (4H, m), 4.81 (1H, d, J 5.0Hz), 4.96 (1H, d, J 5.1Hz) 5.92 (1H, s), 6.99 (1H, s), and 7.25-7.42 (10H, m); [Mass spectrum: +ve ion (3-nitrobenzyl alcohol, sodium acetate) MNa⁺ (533)].

### (c) Diphenylmethyl (6R,7R)-3-[(2,5-Dihydro-2-oxo-thien-4-yl)thiomethyl]-7-[2-(2-tritylaminothiazol-4-yl)-2-(Z) -methoxyiminoacetamido]ceph-3-em-4-carboxylate

A stirred solution of 2-(2-Tritylaminothiazol-4-yl)-2-(Z)-methoxyiminoacetic acid hydrochloride (259mg) in dimethylformamide (4ml) was cooled to -50°C under an inert atmosphere, N,N-Diisopropylethylamine (187µl) and methanesulphonyl chloride (42µl) were added and the reaction stirred at -50°C for 45 min. Pyridine (44µl) and a solution of diphenylmethyl (6R,7R)-7-amino-3-[(2,5-dihydro-2-oxothien-4-yl)thiomethyl]ceph-3-em-4-carboxylate (275mg) in dimethylformamide (3ml) were added and the reaction stirred at 0°C for 1h. The solution was then poured into ethyl acetate/water and the organic extract washed with further portions of water and then brine. After drying over anhydrous magnesium sulphate the crude material was purified by chromatography on silica gel (Kieselgel) eluting with 1:1 ethyl acetate:hexane. The title compound (258mg) was isolated as a foam, νₘₐₓ (CHCl₂) 3400, 1795, 1630, and 1585cm⁻¹; δ_{H}(CDCl₃) 3.42 (1H, d, J 18.3Hz), 3.63 (1H, d, J 18.4Hz) 3.83-4.16 (7H, m), 5.08 (1H, d, J 4.9Hz), 5.88 (1H, s), 5.97 (1H, dd, J 8.8Hz, 4.9Hz) 6.74 (1H, s), 6.79 (1H, bd, J 8.9Hz),6.97 (1H, s), and 7.26-7.39 (25H, m); [Mass spectrum: +ve ion 3-nitrobenzyl alcohol, sodium acetate) MNa⁺ (958)].

### (d) Sodium (6R,7R)-7-[2-(2-Aminothiazol-4-yl)-2-(Z)-methoxyiminoacetamido]-3-[(2,5-dihydro-2-oxothien-4 -yl)thiomethyl]ceph-3-em-4-carboxylate

Diphenylmethyl (6R,7R)-3-[(2,5-dihydro-2-oxothien-4-yl)thiomethyl]-7-[2-(2-tritylaminothiazol-4-yl)-2-(Z)-methoxyiminoacetamido]ceph-3-em-4-carboxylate (258mg) was completely dissolved in 98% formic acid (2.75ml), treated with 1.0M hydrochloric acid (275µl) and stirred at room temperature for 30 min. One drop of concentrated hydrochloric acid was added and stirring continued for 30 min. The reaction mixture was filtered and the solid washed 5 with 90% formic acid. The filtrate was evaporated to dryness and toluene was evaporated from the resulting residue (four times). Ethyl acetate and water were added and the pH adjusted to 6.2 with saturated sodium bicarbonate solution. After separation the aqueous phase was evaporated to low volume and purified by chromatography on HP20SS resin eluting with aqueous mixtures containing increasing proportions of tetrahydrofuran. The resulting aqueous solution was evaporated to low volume and freeze dried, giving the title compound as a white solid (43mg), νₘₐₓ(KBr disc) 1764, 1653, 1533, and 1458cm⁻¹; δ_{H}(D₂O) 3.38 (1H, d, J 17.7Hz), 3.72 (1H, d, J 17.7Hz), 3.88 (1H, d, J 13.5Hz), 3.93 (3H, s), 4.16 (1H, d, J 13,3Hz) 4.25 (2H, s), 5.15 (1H, d, J 4.7Hz), 5.74 (1H, d, J 4.6Hz), 6.19 (1H, s), and 6.97 (1H, s); [mass spectrum: +ve ion (thioglycerol) MH⁺ (550)].

### Example 2

### Pivaloyloxymethyl (6R,7R)-7-[2-(2-Aminothiazol-4-yl)- 2-(Z)-methoxyiminoacetamido]-3-[(2,5-dihydro-2-oxothien-4 -yl)thiomethyl]ceph-3-em-4-carboxylate

Sodium iodide (76mg) was added to a stirred solution of pivaloyloxymethyl bromide in acetone (3ml). The heterogeneous mixture was stirred in the dark for 15 min. before filtering through Kieselguhr. After removing the solvent the residue was triturated with toluene. The toluene phase was filtered into a solution of sodium (6R,7R)-7-[2-(2-aminothiazol-4-yl)-2-(Z)-methoxyiminoacetamido]-3-[(2,5-dihydro-2-oxothien-4-yl)-thiomethyl]ceph-3-em-4-carboxylate (139mg) in N-methyl pyrollidinone (4ml). The reaction was stirred at room temperature for 30 min. Evaporation of the solvent in vacuo gave a residue which was partitioned between ethyl acetate and water. The organic phase was washed with copious amounts of water and then brine before drying over anhydrous magnesium sulphate. 5 Purification was accomplished by chromatography on silica gel (Kieselgel) eluting with 7:3 ethyl acetate:hexane followed by neat ethyl acetate. Solvent was removed giving the title compound (102mg) as an amorphous solid, νₘₐₓ(CH₂Cl₂) 3480, 1785, 1740, and 1680cm⁻¹; δ_{H}(CDCl₃) 1.22 (9H, s), 3.51 (1H, d, J 18.4Hz), 3.66 (1H, d, J 18.4Hz), 3.99 (1H, d, J 12.8Hz), 4.06 (3H, s), 4.19 (1H, d, J 12.7Hz), 5.12 (1H, d, J 4.8Hz), 5.34 (1H, bs, ex. in D₂O), 5.83-6.12 (4H, m), 6.87 (1H, s), and 7.48 (1H, bd, ex. in D₂O, J 8.7Hz); [Mass spectrum: +ve ion (3-nitrobenzyl alcohol, sodium acetate) MNa⁺ (664)].

### Example 3

### Sodium (6R,7R)-7-[2-(2-Aminothiazol-4-yl)-2-(Z)-methoximinoacetamido-3-[(N-methoxy-2-oxopyrrolidin -3-yl)thio]ceph-3-em-4-carboxylate

### (a) Diphenylmethyl (6R,7R)-3-[(N-methoxy-2-oxopyrollidin-3-yl)thio]-7-phenylacetamidoceph-3-em-4 -carboxylate

Diphenylmethyl (6R,7R)-3-mercapto-7-phenylacetamidoceph-3-em-4-carboxylate (774mg) was dissolved in dry tetrahydrofuran (15ml) and treated with 3-bromo-N-methylpyrrolidin-2-one (290mg) and N,N-diisopropylethylamine (260µl). The reaction was stirred for 1h. at room temperature. Solvent was removed by evaporation in vacuo and the residue dissolved in dichloromethane. Chromatography was carried out on silica gel (Kieselgel) eluting with 1:1 ethyl acetate:hexane. The product was then triturated with diethy ether to give the title compound as an off white solid (664mg). Crystallisation was achieved from an ethyl acetate/hexane mixture, (Found: C, 62.99; H, 4.81; N, 6.68; S, 10.11; C₃₃H₃₁N₃O₆S₂, requires; C, 62.94; H, 4.96; N, 6.67; S, 10.18); νₘₐₓ(CH₂Cl₂) 3410, 1785, 1715, and 1595cm⁻¹; δ_{H}(CDCl₃) 1.72-1.88 (1H, m), 2.23-2.37 (1H, m), 3.37-4.05 (7H, m) 3.70 and 3.74 (3H, s+s), 4.98 and 5.01 (1H, d+c, J 4.8Hz), 5.81 (1H, dd, J 9.1Hz, 4.8Hz), 6.24 and 6.36 (1H, d+d, J 9.1Hz), 6.85 and 6.92 (1H, s+s), and 7.26-7.43 (15H, m); [Mass spectrum: +ve ion (3-nitrobenzyl alcohol, sodium acetate) MNa⁺ (652)].

### (b) Diphenylmethyl (6R,7R)-7-Amino-3-[(N-methoxy-2-oxopyrrolidin-3-yl)thio]ceph-3-em-4-carboxylate

Diphenylmethyl (6R,7R)-3-[(N-methoxy-2-oxopyrrolidin-3-yl)thio]-7-phenylacetamidoceph-3-em-4-carboxylate (190mg) was dissolved in dichloromethane (2.4ml) and the solution cooled to -20°C whilst under an inert atmosphere. N-methylmorpholine (66µl) was added followed by a solution of phosphorous pentachloride in dichloromethane (2.34ml of 40mg ml⁻¹ solution). The reaction was stirred at -20°C for 30 min. before adding methanol (600µl) and allowing to warm to room temperature. After stirring for 30 min. water (812µl) was added and vigorous stirring continued for 1h. Solvent was removed by evaporation in vacuo and the residue was dissolved in ethyl acetate and water. The pH was adjusted to 6.5 with 1.0M aqueous ammonia. After separation the organic phase was washed with water and brine and then dried over anhydrous magnesium sulphate. The crude material was purified by chromatography on silica gel (Kieselgel) to give the title compound which was isolated as a foam (88mg), νₘₐₓ(CHCl₂) 3410, 1785, and 1715cm⁻¹; δ_{H}(CDCl₃) 1.79-1.88 (3H, m), 2.08-2.33 (1H, m), 3.30-3.55 (3H, m), 3.68-3.82 and 4.11-4.18 (5H, m), 4.72-4.76 (1H, m), 6.92 and 6.99 (1H, s+s), and 7.26-7.49 (10H, m); [Mass spectrum: +ve ion (3-nitrobenzyl alcohol, sodium acetate) MNa⁺ (534)].

### (c) Diphenylmethyl (6R,7R)-3-[(N-Methoxy-2-oxopyrrolidin-3-yl)thio]-7-[2-(2-tritylaminothiazol-4-yl) -2-(Z)-methoxyiminoacetamido]ceph-3-em-4-carboxylate

A solution of 2-(2-tritylaminothiazol-4-yl)-2-(Z)-methoxyiminoacetic acid hydrochloride (299mg) in dimethylformamide (5ml) was cooled to -50°C whilst under an atmosphere of argon. The solution was treated with N,N-diisopropylethylamine (217µl) followed by methanesulphonyl chloride (49µl). The reaction was stirred for 45 min. at -50°C before treating with pyridine (51µl) followed by a solution of diphenylmethyl (6R,7R)-7-amino-3-[(N-methoxy-2-oxopyrrolidin-3-yl)thio]ceph-3-em-4-carboxylate (320mg) in dimethylformamide (3ml). After allowing to warm to room temperature the reaction was stirred for 15 min. The reaction was poured into ethyl acetate and water. The organic phase was separated, washed with water and brine and dried over anhydrous magnesium sulphate. Purification was accomplished by chromatography on silica gel (Kieselgel) eluting with 7:3 ethyl acetate/hexane. The title compound was finally isolated as a foam (488mg), νₘₐₓ(CH₂Cl₂) 3390, 1790, 1715, and 1515cm⁻¹; δ_{H}(CDCl₃) 1.75-1.88 (1H, m), 2.14-2.18 (1H, m), 3.36-3.86 (8H, m), 4.07 (3H, s), 5.08 and 5.12 (1H, d+d, J 4.76Hz), 5.91 (1H, dd, J 8.82Hz, 4.64Hz), 6.74 and 6.77 (1H, s+s), 6.89-7.09 (3H, m), and 7.19-7.50 (25H, m).

### (d) Sodium (6R,7R)-7-[2-(2-Aminothiazol-4-yl)-2-(Z)-methoxyiminoacetamido]-3-[(N-methoxy-2-oxopyrrolidin-3 -yl)thio]ceph-3-em-4-carboxylate

Diphenylmethyl (6R,7R)-3-[(N-methoxy-2-oxopyrrolidin-3-yl)thio]-7-[2-(2-tritylaminothiazol-4-yl)-2-(Z)-methoxyiminoacetamido]ceph-3-em-4-carboxylate (405mg) was completely dissolved in 98-100% formic acid (4.0ml) before treating with 1.0M hydrochloric acid (410µl). The reaction was stirred for 30 min. at room temperature before adding a drop of concentrated hydrochloric acid. The reaction was stirred for a further 45 min. Any solid was removed by filtration and then washed with 90% formic acid. The filtrate was evaporated to dryness and toluene was evaporated from the residue (three times). After suspending the residue in ethyl acetate and water the pH was adjusted to 6.5 with saturated aqueous sodium bicarbonate solution. The aqueous phase was evaporated to low volume and chromatographed on HP20SS resin eluting with aqueous solutions containing an increasing proportion of tetrahydrofuran. Evaporation to low volume followed by freeze drying gave the title compound as a mixture of diastereoisomers (188mg), νₘₐₓ(KBr disc) 1764, 1670, and 1529cm⁻¹; δ_{H}(D₂O) 2.04-2.15 (1H, m), 2.44-2.53 (1H, m), 3.75 (3H, s), 3.96 (3H, s) superimposed on 3.38-4.05 (5H, m), 5.24 and 5.26 (1H, d+d, J 4.73Hz), 5.76-5.80 (1H, m), and 6.99 (1H, s); [Mass spectum: +ve ion (thioglycerol) MH⁺ (551)].

### Example 4

### Pivaloyloxymethyl (6R,7R)-7-[2-(2-Aminothiazol-4-yl)-2-(Z)-methoxyiminoacetamido]-3-[(N-methoxy-2-oxopyrrolidin-3 -yl)thio]ceph-3-em-4-carboxylate

Sodium (6R,7R)-7-[2-(2-aminothiazol-4-yl)-2-(Z)-methoxyiminoacetamido]-3-[(N-methoxy-2-oxopyrrolidin-3-yl)thio]ceph-3-em-4-carboxylate (164mg) was reacted to give the title compound (71mg) in the manner described in Example 2, νₘₐₓ(CH₂Cl₂) 1785, 1755, and 1735cm⁻¹; δ_{H}(CDCl₃) 1.23 (9H, s), 1,97-2.08 (1H, m), 2.45-2.65 (1H, m), 3.78 (3H, s), 4.06 (3H, s) superimposed on 3.53-4.23 (5H, m), 5.11-5.15 (1H, m), 5.43 (2H, bs) 5.80-6.02 (3H, m), 6.89-6.91 (1H, m), and 7.48-7.52 (1H, m). [Mass spectrum: +ve ion (3-nitrobenzyl alcohol, sodium acetate) MNa⁺ (665)].

### Example 5

### Sodium (6R,7R)-7-[2-(2-Aminothiazol-4-yl-2-(Z)-methoxyiminoacetamido-3-[(N-methyl-2-oxopyrrolidin-3 -yl)thio]ceph-3-em-4-carboxylate

### (a) Diphenylmethyl (6R,7R)-3-[(N-Methyl-2-oxopyrollidin-3-yl)thiol]-7-phenylacetamidoceph-3-em-4-carboxylate

N,N-Diisopropylethylamine (252µl) was added to a mixture of diphenylmethyl (6R,7R)-3-mercapto-7-phenylacetamido-ceph-3-em-4-carboxylate (750mg) and 3-bromo-N-methylpyrrolidin-2-one in tetrahydrofuran (10ml). The reaction was stirred at room temperature for 2hr. Solvent was removed by evaporation in vacuo and the residue suspended in ethyl acetate. Purification was accomplished by chromatography on silica gel (Kieselgel) eluting with ethyl acetate. Trituration of the product gave the title compound as an amorphous solid (768mg), νₘₐₓ(CH₂Cl₂) 3420, 1790, and 11695cm⁻¹; δ_{H}(CDCl₃) 1.73-1.91 (1H, m), 2.25-2.39 (1H, m), 2.79 and 2.82 (3H, s+s), 3.16-3.36 (2H, m), 3.55-3.80 (4H, m), 4.11 and 4.17 (1H, d+d, J 16.5Hz), 4.98 and 5.02 (1H, d+d, J 4.7Hz), 5.79 (1H, dd, J 9.1Hz, 4.7Hz), 6.41 and 6.50 (1H, d+d, J 9.lHz) 6.86 and 6.91 (1H, s+s), and 7.21-7.53 (15H, m); [Mass spectrum: +ve ion (3-nitrobenzyl alcohol, sodium acetate) MNa⁺ (636)].

### (b) Diphenylmethyl (6R,7R)-7-Amino-3-N-methyl-2-oxopyrrolidin-3-yl)thio]ceph-3-em-4-carboxylate

Diphenylmethyl (6R,7R)-3-[(-methyl-2-oxopyrrolidin-3-yl)thio]-7-phenylacetamidoceph-3-em-4-carboxylate (590mg) was dissolved in dichloromethane (7.5ml) and cooled to -20°C whilst under an inert atmosphere. The solution was treated with N-methylmorpholine (211µl) followed by a solution of phosphorous pentachloride in dichloromethane (7.5ml of 40mg ml⁻¹ solution). After stirring at -20°C for 30 min. the reaction was treated with methanol (1.9ml) and the reaction allowed to warm to room temperature stirring was continued for 30 min. before treating with water (2.6ml) and vigorously stirring for 1h. Solvent was removed by evaporation in vacuo and the residue dissolved in ethyl acetate and water. The pH was adjusted to 6.5 with 1.0M aqueous ammonia before separating the organic phase, washing with water and brine and then drying over anhydrous magnesium sulphate. Purification was accomplished by chromatography on silica gel (Kieselgel) employing gradient elution (ethyl acetate followed by 19:1 ethyl acetate/ethanol). The title compound (385mg) was isolated as a foam, νₘₐₓ(CH₂Cl₂) 1780 and 1695cm⁻¹; δ_{H}(CDCl₃) 1.76-1.90 (1H, m), 2.22-2.36 (1H, m) superimposed on 2.40 (2H, bs), 2.79 and 2.81 (3H, s+s), 3.15-3.34 (2H, m), 3.63-4.24 (3H, m), 4.75 (1H, d, J 4.96Hz) 5.01 (1H, d, J 4.93), 6.92 and 6.95 (1H, s+s), and 7.23-7.49 (10H, m); [Mass spectum: +ve ion (3-nitrobenzyl alcohol, sodium acetate) MNa⁺ (518)].

### (c) Diphenylmethyl (6R,7R)-3-[(N-methyl-2-oxopyrrolidin-3-yl)thio]-7-[2-(2-tritylaminothiazol-4-yl)-2-(Z) -methoxyiminoacetamido]ceph-3-em-4-carboxylate

A stirred solution of 2-(2-tritylaminothiazol-4-yl)-2-(Z)-methoxyiminoacetic acid (310mg) in dimethylformamide (4ml) was cooled to -50°C under an inert atmosphere before treating with N,N-diisopropylethylamine (134µl) followed by methanesulphonyl chloride (60µl). The reaction was stirred at -50°C for 45 min. before allowing to warm to -20°C and recooling to -50°C. After treating with ,-diisopropylethylamine (122µl) and a solution of diphenylmethyl (6R,7R)-7-amino-3-[(N-methyl-2-oxopyrrolidin-3-yl)-thio]ceph-3-em-4-carboxylate (348mg) in dimethylformamide (3ml) the reaction was allowed to warm to 0°C and stirred for 30 min. The reaction was partitioned between ethyl acetate and water. After washing the organic phase with water (twice) and brine it was dried over anhydrous magnesium sulphate. Purification by chromatography on silica gel (Kieseigel) employing gradient elution (7:3 ethyl acetate/hexane rising to ethyl acetate) gave the title compound (442mg) finally isolated as a foam, νₘₐₓ(CH₂Cl₂) 3400, 1790, 1735, and 1695cm⁻¹; δ_{H}(CDCl₃) 1.81-1.91 (1H, m), 2.24-2.46 (1H, m), 2.77 and 2.80 (3H, s+s), 3.18-3.38 (2H, m), 3.62-3.79 (2H, m), 4.06 (3H, s) superimposed on 4.06-4.34 (1H, m), 5.04 and 5.09 (1H, d+d, J 4.76Hz), 5.85-5.91 (1H, m) 6.74 and 7.12 (1H, d+d, J 8.74Hz), 6.89 and 6.92 (1H, s+s), 7.01 (1H, bs), and 7.26-7.47 (25H, m); [Mass spectrum: +ve ion (3-nitrobenzyl alcohol, sodium acetate) MNa⁺ (943)].

### (d) Sodium (6R,7R)-7-[2-(2-Aminothiazol-4-yl)-2-(Z)-methoximinoacetamido]-3-[(N-methyl-2-oxopyrrolidin-3 -yl)thio]ceph-3-em-4-carboxylate

Diphenylmethyl (6R,7R)-3-[(-methyl-2-oxopyrrolidin-3-yl)thio)-7-[2-(2-tritylaminothiazol-4-yl)-2-(Z)-methoxylminoacetamizo]ceph-3-em-4-carboxylate (433mg) was completely dissolved in 98-100% formic acid (4.7ml) before treating with 1.OM hydrochloric acid (470µl). After stirring the reaction for 30 min. a drop of concentrated hydrochloric acid was added and stirring continued for 30 min. The solvent was removed by evaporation in vacuo and toluene was evaporated from the residue (three times). The resulting residue was suspended in an ethyl acetate/water mixture and the pH adjusted to 6.5 with saturated aqueous sodium bicarbonate. The aqueous phase was separated, evaporated to low volume, and purified by chromatography on HP20SS resin eluting with aqueous solutions containing an increasing proportion of tetrahydrofuran. The product containing fractions were combined, evaporated to low volume and freeze dried to give the title compound (192mg) as a mixture of diastereoisomers, νₘₐₓ(KBr disc) 1762, 1699, and 1611cm⁻¹; δ_{H}(D₂O) 1.98-2.13 (1H, m), 2.36-2.54 (1H, m), 2.82 (3H, s), 3.38-3.57 (3H, m), 3.71 (1H, d, J 17.3Hz), 3.96 (3H, s) superimposed on 3.78-3.99 (1H, m), 5.23 (1H, d, J 4.7Hz), 5.78 (1H, d, J 4.76Hz), and 6.96 (1H, s); [Mass spectrum: +ve ion (thioglycerol) MH⁺ (535)].

### Example 6

### Sodium (6R,7R)-7-[2-(Aminothiazol-4-yl)-2-(Z)-methoxyiminoacetamido]-3-[(2-oxotetrahydrothien-3 -yl)thio]ceph-3-em-4-carboxylate

### (a) Diphenylmethyl (6R,7R)-3-[(2-Oxotetrahydrothien-3-yl)thio]-7-Phenylacetamidoceph-3-em-4-carboxylate

N,N-Diisopropylethylamine (336µl) was added to a mixture of diphenylmethyl (6R,7R)-3-mercapto-7-phenylacetamidoceph-3-em-4-carboxylate (1.00g) and 3-bromotetrahydrothiophene-2-one (350mg) in tetrahydrofuran (15ml). The reaction was stirred at room temperature for 30 min. Solvent was removed by evaporation in vacuo and the resulting residue dissolved in ethyl acetate and water. Precipitation of a solid occurred. This was removed by filtration and the entire filtrate was evaporated to dryness. The collected solid and the filtrate residue were dissolved together in hot acetone and the solution then allowed to cool. Any precipitated solid was collected by filtration to give the title compound as an off white solid (660mg), νₘₐₓ(CHCl₃) 3410, 1790, and 1690cm⁻¹; δ_{H}(CDCl₃) 1.94-2.10 and 2.29-2.49 (2H, m) 3.16-3.33 (2H, m), 3.45-3.79 (5H, m), 4.98-5.02 (1H, m), 5.78-5.85 (1H, m), 6.16 and 6.26 (1H, d+d, J 9Hz), 6.90 and 6.98 (1H, s+s), and 7.21-7.42 (15H, m); [Mass spectrum: +ve ion (3-nitrobenzyl alcohol, sodium acetate) MNa⁺ (639)].

### (b) Diphenylmethyl (6R,7R)-7-Amino-3-[(2-oxotetrahydrothien-3-yl)thio]ceph-3-em-4-carboxylate

Diphenylmethyl (6R,7R)-3-[(2-oxotetrahydrothien-3-yl)thio]-7-phenylacetamidoceph-3-em-4-carboxylate (660mg) was suspended in dichloromethane (10ml). N-methylmorpholine (221µl) was added and the reaction cooled to -20°C. A solution of phosphorus pentachloride in dichloromethane (8.0ml of 40mg ml⁻¹ solution) was addedand the reaction stirred at -20°C until it washomogeneous. Methanol (2.0ml) was added and the solution allowed to warm to room temperature. After 30 min. water (2.7ml) was added and vigorous stirring was continued for a further 1h. Dichloromethane was removed by evaporation in vacuo and the residue diluted with ethyl acetate/water. The pH was adjusted to 6.5 with 1.0M aqueous ammonia before separating the organic phase, washing with brine and drying over anhydrous magnesium sulphate. Chromatography was carried out on silica gel (Kieselgel) employing gradient elution (1:1 ethyl acetate:hexane rising to 7:3 ethyl acetate hexane) which gave the title compound as a 1:1 mixture of diastereomers (355mg), νₘₐₓ(CH₂Cl₂) 1780, 1730, and 1700cm⁻¹; δ_{H}(CDCl₃) 1.85 (2H, bs), 2.0-2.4 (2H, m), 3.0-3.4 (2H, m), 3.6-4.2 (3H, m), 4.70 (1H, d, J 5Hz), 4.9(1H, d, J 5Hz), 6.9 and 7.0 (1H, s+s), and 7.2-7.5 (10H, m); [Mass spectrum: +ve ion (3-nitrobenzyl alcohol, sodium acetate) MNa⁺ (521)].

### (c) Diphenylmethyl (6R,7R)-3-[(2-Oxotetrahydrothien-3-yl)thio]-7-[2-(2-tritylaminothiazol-4-yl)-2-(Z) -methoxyiminoacetamido]ceph-3-em-4-carboxylate

A stirred solution of 2-(2-tritylaminothiazol-4-yl)-2-(Z)-methoxyiminoacetic acid hydrochloride (341mg) in dimethylformamide (5ml) was treated with N,N-diisopropylethylamine (123µl) and then cooled to -50°C whilst under an inert atmosphere. Methanesulphonyl chloride (55µl) was added and the reaction stirred at -50°C for 45 min. A solution of diphenylmethyl (6R,7R)-7-amino-3-[(2-oxotetrahydrothien-3-yl)thioceph-3-em-4-carboxylate (355mg) in dimethylformamide (2ml) was added followed by pyridine (56µl). The reaction was allowed to warm to 0°C and stirred for 15 min. After pouring the reaction mixture into ethyl acetate/water the organic phase was washed sequentially with saturated sodium bicarbonate solution, water, and brine. The solution was dried over anhydrous magnesium sulphate before removing the solvent with a rotary evaporator. The residue was dissolved in dichloromethane and subjected to chromatography on silica gel (Kieselgel) eluting with 1:1 ethyl acetate:hexane rising to neat ethyl acetate. The title compound was finally isolated as a mixture of diastereomers (411mg), νₘₐₓ(CH₂Cl₂) 3390, 1785, 1730, and 1690cm⁻¹; δ_{H} (CDCl₃) 1.97-2.15 and 2.30-2.53 (2H, m), 3.13-3.40 (2H, m), 3.53-4.16 (3H, m), 4.08 (3H, s), 5.09 and 5.11 (1H, d+d, J 4.0Hz), 5.88-5.97 (1H, m), 6.67-6.80 (2H, m), 6.94 and 7.0 (1H, s+s), 7.01 (1H, bs), and 7.26-7.51 (25H, m); [Mass spectrum: +ve ion (3-nitrobenzyl alcohol, sodium acetate) MNa⁺ (946)].

### (d) Sodium (6R,7R)-7-[2-(2-Aminothiazol-4-yl)-2-(Z)-methoxyiminoacetamido]-3-[(2-oxotetrahydrothien-3 -yl)thioceph-3-em-4-carboxylate

Diphenylmethyl (6R,7R)-3-[(2-oxotetrahydrothien-3-yl)thio]-7-[2-(2-tritylaminothiazol-4-yl)-2-(Z)-methoxyiminoacetamido]ceph-3-em-4-carboxylate (411mg) was completely dissolved in 98-100% formic acid (4.4ml) before 5 treating with 1.0M hydrochloric acid (440µl). The reaction was stirred at room temperature for 30 min. before treating with one drop of concentrated hydrochloric acid. Stirring was continued for a further 30 min. The solution was filtered and the solid washed with a small amount of 90% formic acid. The filtrate was evaporated to dryness and toluene was evaporated from the residue (three times). The residue was dissolved in ethyl acetate/water and the pH adjusted to 6.5 with saturated sodium bicarbonate. The aqueous phase was separated, evaporated to low volume and chromatographed on HP20SS resin eluting with water containing an increasing proportion of tetrahydrofuran. The product was freeze dried giving the title compound as a mixture of diastereoisomers (139mg), νₘₐₓ(KBr disc) 1764, 1670, 1611, and 1528cm⁻¹; δ_{H}(D₂O) 2.20-2.30 (1H, m), 3.33-3.61 (3H, m), 3.77 and 3.84 (1H, d+d, J 7.7Hz), 3.95 (3H, s), superimposed on 3.90-3.98 (1H, m) 5.21-5.24 (1H, m), 5.78 (1H, d, J 4.7Hz), and 6.98 (1H, s); [Mass spectrum: +ve ion (thioglycerol) MH⁺ (538), MNa⁺ (560)].

### Example 7

### Pivaloyloxymethyl (6R,7R)-7-[2-(2-Aminothiazol-4-yl)-2-(Z)-methoxyiminoacetamido]-3-[(2-oxotetrahydrothien-3 -yl)thiolceph-3-em-4-carboxylate

Sodium (6R,7R)-7-[2-(2-aminothiazol-4-yl)-2-(Z)-methoxyiminoacetamido]-3-[(2-oxotetrahydrothien-3-yl)thio]ceph-3-em-4-carboxylate (115mg) was reacted to give the title compound (32mg) in a manner described in Example 2, νₘₐₓ(CH₂Cl₂) 1785, 1760, and 1680cm⁻¹; δH(CDCl₃) 1.23 (9H, s), 2.25-2.34 (1H, m), 2.61-2.72 (1H, m), 3.25-3.62 (1H, m), 3.69-3.71 (1H, m), 3.91-4.13 (5H, m), 5.11-5.15 (1H, m), 5.27 (2H, bs), 5.86-6.03 (3H, m), 6.92 (1H, s+s), and 7.31 (1H, m); [Mass spectrum: +ve ion (thioglycerol) MH⁺ (630)].

### Example 8

### Sodium (6R,7R-7-[2-(2-aminothiazol-4-yl)-2-(Z)-methoxyiminoacetamido]-3-(2-oxotetrahydrothien-5-yl)ceph-3 -em-4-carboxylate

### (a) (RS)-2-Oxotetrahydrothien-5-ylcarboxylic acid

Sodium hydroxide (9.0g, 225mmol) in water (100ml) was added to dimethyl (RS)-2-acetylthioglutarate (11.99g, 51mmol) (cf. J.B. Fraser, L.N. Owen and G. Shaw, Biochem.J., 1947, 41, 328) followed by industrial methylate spirit (50ml) to give a homogeneous solution which was stirred overnight. The solution was concentrated in vacuo and the residue added to wet Amberlite IR120(H⁺) (125ml) then passed through a column of IR120(H⁺). The aqueous eluent was evaporated to give the title compound as a semi-crystalline gum (7.00g). This in acetone was dried (MgSO₄) and evaporated to give 3-(2,2-dimethyl-3-oxatetrahydrothien-4-yl)propionic acid (7.586g, 73%); δ_{H} [(CD₃)₂CO, 90MHz] 1.70 (6H, bs), 1.9-2.8 (4H, m), 3.4-3.8 (1H, m), 10.18 (1H, bs, exch.). [Mass spectrum: M⁺ (204.1)]. This acetone adduct (7.586g) and toluene-4-sulphonic acid (250mg) were heated in an oil bath at 140°C under a stream of argon for 1.5h. 5 The resulting gum in dichloromethane was washed with water, then the aqueous wash extracted three times with ethyl acetate. The combined organic phases were dried and evaporated to give the title compound as an oil (6.227g, 84%), (Found: M⁺, 146.0038. C₅H₆SO₃ requires: M, 146.0038); νₘₐₓ (CH₂Cl₂) 3500-2500, 1715, 1415 and 1073cm⁻¹; δ_{H} (CDCl₃, 250MHz) 2.4-2.7 (3H, m), 2.8-3.0 (1H, m), 4.49 (1H, dd, J 4.5, 6.6Hz) and 8.41 (1H, bs, exch.).

### (b) (RS)-5-Chloroacetyltetrahydrothien-2-one

(RS)-5-Oxotetrahydrothien-2-ylcarboxylic acid (6.227g, 43mmol) in dichloromethane (50ml) was treated with oxalyl chloride (5.6ml, 64.5mmol) and DMF (1 drop) and stirred 1h then evaporated to dryness in vacuo. The residue was dissolved in dichloromethane and reevaporated to give the acid chloride, νₘₐₓ (CH₂Cl₂) 1776 and 1723cm⁻¹.

The acid chloride in ether (100ml) was added dropwise to an ice bath cooled solution of diazomethane (ca 100mmol) in ether (200ml). When the addition was completed the mixture was stirred 0.25h, νₘₐₓ (film) 2110 and 1711cm⁻¹, then a stream of hydrogen chloride gas passed into the mixture for 5 minutes. After a further 0.25h the mixture was washed twice with brine, dried, concentrated and flash chromatographed on silica gel eluting with 20, 25% ethyl acetate in hexane to give the title compound (5.531g, 72%); (Found: M⁺, 177.9864. C₆H₇ClSO₂ requires M, 177.9855); νₘₐₓ (CH₂Cl₂) 1720, 1062 and 909cm⁻¹; δ_{H} (CDCl₃, 250MHz) 2.3-2.45 (1H, m), 2.55-2.9 (3H, m), 4.23 and 4.32 (2H, ABq, J 15.5Hz) and 4.80 (1H, dd, J 3.1, 6.4Hz).

### (c) t-Butyl (RS)-2-hydroxy-2-[(3R,4R)-4-[(RS)-2-oxotetrahydrothien-5-ylcarbonylmethylthio-3 -phenoxyacetamidoazetidin-2-on-1-yl]acetate.

Toluene-4-sulphonic acid (8.335g) in water (20ml) was added to t-butyl (RS)-2-hydroxy-2-[(1R,5R)-3-phenoxymethyl-2,6-diaza-4-thiabicyclo[3.2.0]hept-2-en-7-on-6-yl]acetate (9.100g, 25mmol) (See EP-A-O 395 219)in acetone (40ml) and dichloromethane (40ml) under argon then stirred 1h. The solution was diluted with dichloromethane, washed with water, dried and evaporated to give crude t-butyl (RS)-2-hydroxy-2-[(3R,4R)-4-mercapto-3-phenoxyacetamidoazetidin-2-on-1-yl]acetate as a foam (9.705g). To this in acetone (10ml) under argon was added (RS)-5-chloroacetyltetrahydrothien-2-one (4.878g, 27.25mmol) in acetone (5ml) followed by potassium carbonate (1.725g, 12.5mmol). The mixture was stirred 0.5h, diluted with ethyl acetate, washed twice with water and with brine, dried, concentrated and flash chromatographed on silica gel eluting with 25,30,35,40, 50,60,70 and 80% ethyl acetate in hexane to give the title compound as a foam (8.605g, 66%); νₘₐₓ (CH₂Cl₂) 3400, 1784, 1732, 1694, 1520, 1288, 1240 and 1155cm⁻¹; δ_{H} (CDCl₃, 250MHz) 1.52, 1.54 (together 9H, 2s), 2.15-2.85 (4H, m), 3.4-3.65 (2H, m), 4.59 (2H, s), 4.4-5.6 (5H, m), 6.85-7.4 (5H, m) and 7.35, 7.43, 7.52, 7.60 (together 1H, 4d, J 8.6Hz); [Mass spectrum: +ve ion (3-nitrobenzyl alcohol, sodium acetate) MNa⁺ (549)].

### (d) t-Butyl 2-[(3R,4R)-4-[(RS)-2-oxotetrahydrothien-5-ylcarbonylmethylthio]-3-phenoxyacetamidoazetidin-2-on-1-yl] -2-tri-n-butylphosphoranylideneacetate.

Thionyl chloride (1.8ml, 24.6mmol) in THF (10ml) was added dropwise to a solution of the hydroxy compound (8.605g, 16.4mmol) and 2,6-lutidine (2.86ml, 24.6mmol) in THF (30ml) at ca -20°C. The mixture was stirred 0.5h. without further cooling, filtered and the filtrate evaporated in vacuo, toluene added and reevaporated to give crude t-butyl (RS)-2-chloro-2-[(3R,4R)-4-[(RS)2-oxotetrahydrothien-5-ylcarbonylmethylthio]-3-phenoxyacetamidoazetidin-2-on-1-yl]acetate as a foam (9.176g). To this in dioxan (25ml) cooled in an ice bath was added tri-n-butylphosphine (9.0ml, 36.1mmol). The reaction mixture was stirred 0.5h., diluted with ethyl acetate, washed with dilute sodium bicarbonate solution, water and brine, concentrated and flash chromatographed on silica gel eluting with 30,40,50,60 and 70% ethyl acetate in hexane on silica gel eluting with 5 30,40,50,60 and 70% ethyl acetate in hexane to provide the title compound as a foam (4.496g, 39%); νₘₐₓ (CH₂Cl₂) 3416, 1765, 1692, 1626, 1521, 1290, 1241, 1171 and 1059cm⁻¹; [Mass spectrum: +ve ion (3-nitrobenzyl alcohol, sodium acetate) MNa⁺ (731)]

### (e) t-Butyl (6R,7R)-3-[(R and S)-2-oxotetrahydrothien-2-yl]-7-phenoxyacetamidoceph-3-em-4-carboxylate

The phosphorane (4.496g) and benzoic acid (ca 20mg) in toluene (90ml) were purged with argon then heated in an oil bath at 125°C for 8h. The brown solution was allowed to cool, concentrated and flash chromatographed on silica gel eluting with 25,30% ethyl acetate in hexane to give t-butyl (6R,7R)-3-[(S)-2-oxotetrahydrothien-2-yl]-7-phenoxyacetamidoceph-3-em-4-carboxylate (0.854g, 27%); νₘₐₓ (CH₂Cl₂) 3405, 1788, 1709, 1518, 1495, 1370 and 1154cm⁻¹; δ_{H} (CDCl₃, 250MHz) 1.56 (9H, s), 1.9-2.1 (1H, m), 2.6-2.75 (3H, m), 3.50 and 3.54 (2H, ABq, J 18.2Hz), 4.58 (2H, s), 5.01 (1H, d, J 4.9Hz), 5.47 (1H, dd, J 5.2, 10.8Hz), 5.95 (1H, dd, J 4.9, 9.3Hz) and 6.9-7.4 (6H, m). [Mass spectrum: +ve ion (thioglycerol) MH⁺ (491)]; further elution with 35% ethyl acetate in hexane gave t-butyl (6R,7R)-3-[(RS)-2-oxotetrahydrothien-2-yl]-7-phenoxy-acetamidoceph-2-em-4-carboxylate (0.585g, 19%); νₘₐₓ (CH₂Cl₂) 3401, 1782, 1733, 1700, 1518, 1495, 1372 and 1149cm⁻¹; δ_{H} (CDCl₃, 250MHz) 1.52 (9H, s), 2.2-2.8 (4H, m), 4.58 (2H, s), 4.66 (1H, dd, J 5.5, 9.0Hz), 4.88, 4.90 (together 1H, 2d, J 1.1Hz), 5.32, 5.35 (together 1H, 2d, J 4.0Hz), 5.74 (1H, dd, J 4.0, 9.05Hz), 6.46, 6.55 (together 1H, 2bs) and 6.9-7.45 (6H, m). [Mass spectrum: +ve ion (thioglycerol) MH⁺ (491)]; and further elution with 35 and 40% ethyl acetate in hexane gave t-butyl (6R,7R)-3-[(R)-2-oxotetrahydrothien-2-yl]-7-phenoxyacetamidoceph-3-em-4-carboxylate (0.657g, 21%); νₘₐₓ (CH₂Cl₂) 3402, 1790, 1705, 1518, 1495, 1370 and 1154cm⁻¹; δ_{H} (CDCl₃, 250MHz) 1.57 (9H, s), 2.1-2.35 (2H, m), 2.55-2.8 (2H, m), 3.51 and 3.64 (2H, ABq, J 17.5Hz), 4.57 (2H, s), 5.05 (1H, d, J 5.0Hz), 5.43 (1H, dd, J 5.8, 10.7Hz), 5.92 (1H, dd, J 5.0, 9.3Hz) and 6.9-7.4 (6H, m); [Mass spectrum: +ve ion (thioglycerol) MH⁺ (491).

### (f) t-Butyl (6R,7R)-3-[(RS)-2-oxotetrahydrothien-5-yl]-7-phenoxyacetamidoceph-3-em-4-carboxylate-1-oxide

3-Chloroperoxybenzoic acid (0.414g, 55%, 1.32mmol) was added in portions to t-butyl (6R,7R)-3-[(RS)-2-oxotetrahydrothien-2-yl]-7-phenoxyacetamidoceph-2-em-4-carboxylate (0.647g, 1.32mmol) in dichloromethane (10ml) cooled in an ice bath. 0.5h after the final addition the reaction mixture was washed with dilute sodium metabisulphite then with dilute sodium hydrogen carbonate, dried and evaporated to give the title compound as a solid (0.694g); νₘₐₓ (CH₂Cl₂) 3383, 1801, 1707, 1153, 1105 and 1069cm⁻¹. [Mass spectrum: +ve ion (3-nitrobenzyl alcohol, sodium acetate) MNa⁺ (529)].

### (g) t-Butyl (6R,7R)-3-[(R and S)-2-oxotetrahydrothien-5-yl]-7-phenoxyacetamidoceph-3-em-4-carboxylate

Phosphorus trichloride (210µl, 2.64mmol) was added to the sulphoxide from Example 8(f) (0.694g) in DMF (2ml) at -20°C. The reaction was stirred at -20°C for 10 minutes then quenched by the addition of ice followed by ethyl acetate. The organic phase was washed twice with water and with brine, dried, concentrated and flash chromatographed on silica gel eluting with 30, 35, 40 and 50% ethyl acetate in hexane to give the (S) (0.147g, 23%) then the (R) (0.348g, 54%) diastereoisomer of the title compound identical to the materials described in Example 8(e).

### (h) t-Butyl (6R,7R)-7-amino-3-[(S)-2-oxotetrahydrothien-5-yl]ceph-3-em-4-carboxylate

Phosphorus pentachloride (0.697g, 3.4mmol) in dichloromethane (17.4ml) was added to t-butyl (6R,7R)-3-[(S)-2-oxotetrahydrothien-5-yl]-3-phenoxyacetamidoceph-3-em-4-carboxylate (1.110g, 2.26mmol) and N-methylmorpholine (500µl, 4.52mmol) in dichloromethane (25ml) at -25°C. Stirred 0.75h at -10±5°C then methanol (5ml) added, stirred 0.75h then water (20ml) added and the mixture stirred vigorously for 1h. The dichloromethane was removed in vacuo, the aqueous residue washed with ether then adjusted to pH7 with aqueous ammonia in the presence of ethyl acetate. The mixture was extracted twice with ethyl acetate then the combined extracts dried, concentrated and flash chromatographed on silica gel eluting with 50% ethyl acetate in hexane to yield the title compound as a foam (0.343g, 43%); (Found: M⁺, 356.0861. C₁₅H₂₀N₂O₄S₂ requires M, 356.0865); νₘₐₓ (CH₂Cl₂) 1779, 1730(sh), 1710, 1253 and 1155cm⁻¹; δ_{H} (CDCl₃, 250MHz) 1.56 (9H, s), 1.70 (2H, br s), 1.9-2.15 (1H, m), 2.6-2.75 (3H, m), 3.54 and 3.56 (2H, ABq, J 18.4Hz), 4.79 (1H, d, J 5.1Hz), 4.93 (1H, d, J 5.1Hz) and 5.4-5.5 (1H, m).

### (i) t-Butyl (6R,7R)-7-amino-3-[(R)-2-oxotetrahydrothien-5-yl]ceph-3-em-4-carboxylate

Prepared from t-butyl (6R,7R)-3-[(R)-2-oxotetrahydrothien-5-yl]-7-phenoxyacetamidoceph-3-em-4-carboxylate, by the method described in Example 8(h), in 42% yield; (Found: M⁺, 356.0861. C₁₅H₂₀N₂O₄S₂ requires M, 356.0865); νₘₐₓ (CH₂Cl₂) 1783, 1731(sh), 1708, 1370, 1156 and 1045cm⁻¹; δ_{H} (CDCl₃, 250MHz) 1.56 (9H, s), 1.82 (2H, br s), 2.1-2.35 (2H, m), 2.55-2.75 (2H, m), 3.50 and 3.64 (2H, ABq, J 17.5Hz), 4.76 (1H, d, J 5.1Hz), 4.97 (1H, d, J 5.1Hz) and 5.35 (1H, dd, J 5.9, 10.6Hz).

### (j) t-Butyl (6R,7R)-7-[2-(Z)-methoxyimino-2-(2-tritylaminothiazol-4-ylacetamido]-3-[(S)-2-oxo tetrahydrothien-5-yl]ceph-3-em-4-carboxylate

Methanesulphonyl chloride (93µl, 1.2mmol) was added to 2-(Z)-methoxyimino-2-(2-tritylaminothiazol-4-yl)acetic acid hydrochloride (0.417g, 1,1mmol) and N,N-diisopropylethylamine (383µl, 2.2mmol) in DMF (2.5ml) at -40°C. Stirred at -20±10°C for 0.5h then treated with the product from Example 8(h) (0.340g, 0.96mmol) in DMF (2ml) followed by pyridine (98µl, 1.2mmol). The mixture was stirred lh, diluted with ethyl acetate, washed twice with water and with brine, dried, concentrated and flash chromatographed on silica gel eluting with 25, 30 and 40% ethyl acetate in hexane to give the title compound (0.400g, 54%); νₘₐₓ (CH₂Cl₂) 3395, 1788, 1731, 1712, 1519, 1370, 1154 and 1048cm⁻¹; δ_{H} (CDCl₃, 250MHz) 1.56 (9H, s), 1.9-2.1 (1H, m), 2.55-2.75 (3H, m), 3.50 and 3.54 (2H, ABq, J 18.3Hz), 4.08 (3H, s), 5.04 (1H, d, J 4.9Hz), 5.4-5.55 (1H, m), 5.97 (1H, dd, J 4.8, 9.0Hz), 6.73 (1H, d, J 9.0Hz), 6.74 (1H, s), 7.01 (1H, s) and 7.30 (15H, s). [Mass spectrum: +ve ion (thioglycerol) MH⁺ (782)].

### (k) t-Butyl (6R,7R)-7-[2-(Z)-methoxyimino-2-(2-tritylaminothiazol-4-yl)acetamido-3-[(R)-2 -oxotetrahydrothien-5-yl]ceph-3-em-4-carboxylate

Prepared from the product of Example 8(i) using the method described in Example 8(g) in 71%; νₘₐₓ (CH₂Cl₂) 3395, 3283, 1789, 1731, 1709, 1525, 1372, 1154, 1104 and 1046cm⁻¹; δ_{H} (CDCl₃, 250MHz) 1.55 (9H, s), 2.1-2.35 (2H, m), 2.6-2.75 (2H, m), 3.51 and 3.65 (2H, ABq, J 17.6Hz), 4.07 (3H, s), 5.08 (1H, d, J 4.9Hz), 5.43 (1H, dd, J 5.7, 10.7Hz), 5.93 (1H, dd, J 4.9, 8.9Hz), 6.71 (1H, s), 6.79 (1H, d, J 8.9Hz), 7.02 (1H, s) and 7.30 (15H, s). [Mass spectrum: +ve ion (3-nitrobenzyl alcohol, sodium acetate) MNa⁺ (804)].

### (l) Sodium (6R,7R)-7-[2-(2-aminothiazol-4-yl)-2-(Z)-methoxyiminoacetamido]-3-[(S)-2-oxotetrahydrothien-5-yl]ceph-3-em-4-carboxylate

The product from Example 8(j) (0.400g, 0.5lmmol) was dissolved in 0.1M hydrochloric acid in 90% formic acid (5ml) and left 0.5h. Concentrated hydrochloric acid (100µl) was added and the mixture left for a further 1h then evaporated in vacuo and the residue in water adjusted to pH7 with aqueous sodium hydroxide and chromatographed on HP20SS eluting with 0, 1, 2 and 3% THF in water. The fractions containing the title compound (HPLC) were combined concentrated and freeze-dried (187mg, 72%); νₘₐₓ (KBr) 1764, 1674, 1605, 1529, 1388 and 1037cm⁻¹; δ_{H} (d₆ DMSO, 250MHz) 2.0-2.2 (1H, m), 2.3-2.45 (1H, m), 2.55-2.75 (2H, m), ca. 3.5-3.8 (2H, obscured by H₂O/HOD), 3.86 (3H, s) 5.01 (1H, d, J 4.6Hz), 5.58 (1H, dd, J 4.7, 8.0Hz, collapses to d, J 4.6Hz on exch.), 5.69 (1H, d, J 5.5, 10.4Hz), 6.77 (1H, s), 7.25 (2H, s, exch.) and 9.55 (1H, d, J 8.0Hz). [Mass spectrum: +ve ion (thioglycerol) MH⁺ (506), MNa⁺ (528)].

### (m) Sodium (6R,7R)-7-[2-(2-aminothiazol-4-yl)-2-(Z)-methoxyiminoacetamido]-3-[(R)-2-oxotetrahydrothien-5 -yl]ceph-3-em-4-carboxylate

Prepared from the product from Example 8(k) by the method described in Example 8(1), to give the title compound in 78% yield; νₘₐₓ (KBr) 1763, 1675, 1603, 1528, 1389 and 1038cm⁻¹; δ_{H} (9d₆ DMSO, 250MHz) 2.0-2.25 (2H, m), 2.5-2.75 (2H, m), 3.38 and 3.54 (2H, ABq, J 16.8Hz), 3.83 (3H, s), 5.04 (1H, d, J 4.7Hz), 5.55 (1H, dd, J 4.7, 8.1Hz, collapses to d, J 4.8Hz on exch.), 5.68 (1H, dd, J 6.6, 9.6Hz), 6.74 (1H, s), 6.94 (2H, s, exch.) and 9.53 (1H, d, J 8.1Hz). [Mass spectrum: +ve ion (thioglycerol) MH⁺ (506), MNa⁺ (528)].

### EXAMPLE 9

### Sodium (6R,7R)-7-[2-(2-Aminothiazol-4-yl)-2-(Z)-methoxyiminoacetamido]-3-[(2,5-dihydro-2-oxothien-4 -yl)thio]ceph-3-em-4-carboxylate.

### (a) Diphenylmethyl (6R,7R)-3-[(2,5-Dihydro-2-oxothien-4-yl)thio]-7-phenylacetamidoceph-3-em-4-carboxylate.

Diphenylmethyl (6R,7R)-7-phenylacetamido-3-trifluoromethylsulphonylceph-3-em-4-carboxylate (988mg) (V. Farina, S.R. Baker & S.I. Hanck; J. Org. Chem., 1989, 54, 4962) was completely dissolved in dry tetrahydrofuran (10ml) along with (2,5-dihydro-4-mercapto)thien-2-one (288mg) (c.f. Example 1). The mixture was treated with N,N-dispropylethylamine (290µl) and then stirred at room temperature for 2 h. The solvent was then removed by evaporation in vacuo and the residue dissolved in ethyl acetate and water. After washing with a further quantity of water and brine the organic phase was dried over anhydrous magnesium sulphate. Chromatography on silica gel (Kieselgel) eluting with 2:3 ethyl acetate/hexane gave the title compound which was finally isolated as a foam. (685mg); νₘₐₓ(CH₂Cl₂) 3420, 1790, 1735, and 1680 cm⁻¹; δ_{H}(CDCl₃) 3.33(1H,d,J 18.27 Hz) 3.48-3.76 (4H,m), 3.87 (1H,dd, J 18.31, 1.23 Hz), 5.05 (1H,d,J 5.12 Hz) 5.92-5.96 (2H,m), 6.21 (1H,d, J 9.05 Hz), 7.04 (1H,s), and 7.26-7.51 (15H,m). [Mass Spectrum : +ve ion (3-nitrobenzyl alcohol, sodium acetate) MNa⁺ (937)].

### (b) Diphenylmethyl(6R,7R)-7-Amino-3-[(2,5-dihydro-2-oxothien-4-yl)thio]ceph-3-em-4-carboxylate.

Diphenylmethyl (6R,7R)-3-[(2,5-dihydro-2-oxothien-4-yl)thio]-7-phenylacetamidoceph-3-em-4-carboxylate (400mg) was dissolved in dry dichloromethane (5ml) before cooling to -20°C under an atmosphere of argon. N-Methylmorpholine (140µl) and a solution of phosphorus pentachloride in dichloromethane (5.1ml of 40mg ml⁻¹ solution) were added and the reaction stirred at -20°C for 30min. Methanol (1.3ml) was added and the reaction stirred at room temperature for 30 min before adding water (1.75ml) and vigorously stirring for 1h. Solvent was removed by evaporation in vacuo and the residue dissolved in ethyl acetate/water. After adjusting the pH to 6.5 with 1.0M aqueous ammonia the organic phase was washed with brine and dried over anhydrous magnesium sulphate. Flash chromatography on silica gel (Kieselgel) eluting with 1:1 ethyl acetate/hexane rising to 7:3 ethyl acetate/hexane gave the title compound which was finally isolated as a foam (102mg). νₘₐₓ(CH₂Cl₂) 1790, 1735, and 1675cm⁻¹;δ_{H}(CDCl₃) 1.72(2H,bs), 3.35(1H,d,J 18.1 Hz), 3.55(1H,dd,J 18.1Hz, 1.21Hz), 3.70(1H,d,J 18.1Hz), 3.87(1H,dd,J 18.1Hz, 1.3Hz), 4.86(1H,d,J 5.3Hz), 5.04(1H,d,J 5.3Hz), 5.96(1H,s), 7.06(1H,s) and 7.32-7.35(10H,m). [Mass spectrum : +ve ion (3-nitrobenzyl alcohol, sodium acetate) MNa⁺ (519)].

### (c) Diphenylmethyl (6R,7R)-3-[(2,5-Dihydro-2-oxothien-4-yl)thio]-7-[2-(2-tritylaminothiazol-4-yl)-2-(Z) -methoxyiminoacetmido]ceph-3-em-4-carboxylate.

A stirred solution of 2-(2-Tritylaminothiazol-4-yl)-2-(Z)-methoxyiminoacetic acid hydrochloride (94mg) in dimethylformamide (2ml) was cooled to -50°C under an inert atmosphere. N,N-Disopropylethylamine (75µl) and methanesulphonyl chloride (17µl) were added and the reaction stirred at -50° for 40 min. A further portion of N,N-diisopropylethylamine (34µl) was added followed by a solution of diphenylmethyl (6R,7R)-7-amino-3-[(2,5-dihydro-2-oxothien-4-yl)thio]ceph-3-em-4-carboxylate (98mg) in dimethylformamide (2ml). The reaction was allowed to warm to 0°C and stirred for 15 min. The solution was poured into ethyl acetate/water and the organic phase washed with water (twice) and finally brine. After drying over anhydrous magnesium sulphate the material was subjected to flashchromatography on silica gel (Kieselgel) eluting with 3:7 ethyl acetate/hexane rising to 7:3 ethyl acetate/hexane. The flash chromatography was repeated to accomplish complete purification before isolating the title compound as a foam (76mg). νₘₐₓ(CH₂Cl₂) 3400, 1795, 1735, and 1680cm⁻¹; δ_{H}(d₆-acetone) 3.61 and 4.01 (2H,ABq,J, 17.8Hz), 3.92(3H,s) superimposed on 3.88-3.92(1H,m), 4.12(1H,dd,J,18.5Hz,1.4Hz), 5.44(1H,d,J 5.0Hz), 5.94(1H,s), 6.06(1H,dd,J 8.4Hz, 5.0Hz), 6.82(1H,s), 7.04(1H,s), 7.30-7.47(26H,m) and 8.66(1H,d,J 8.3Hz).
[mass spectrum : +ve ion (3-nitrobenzyl alcohol/sodium acetate) MNa⁺ (944); (thioglycerol) MH⁺ (922)].

### (d) Sodium (6R,7R)-7-[2-(2-Aminothiazol-4-yl)-2-(Z)-methoxyiminoacetamido]-3-[(2,5-dihydro-2-oxothien-4 -yl)thio]ceph-3-em-4-carboxylate.

Diphenylmethyl (6R,7R)-3-[(2,5-dihydro-2-oxothien-4-yl)thio]-7-[2-(2-tritylaminothiazol-4-yl)-2-(Z)-methoxyiminoacetamido]ceph-3-em-4-carboxylate (78mg) was completely dissolved in 98-100% formic acid (800µl) before treating with 1.0M hydrochloric acid (84µl) and stirring at room temperature for 40min. A further portion of 1.0M hydrochloric acid (84µl) was added and stirring continued for 30min. The solvents were removed by evaporation in vacuo and toluene was evaporated from the residue (three times). The residue was suspended in ethyl acetate/water and the pH adjusted to 6.3 with saturated sodium bicarbonate solution. The aqueous phase was separated, evaporated to low volume and purified by chromatography on HP20SS resin eluting with aqueous mixtures containing an increasing proportion of tetrahydrofuran. The product containing fractions were combined, evaporated to low volume and freeze dried giving the title compound as a white solid (19mg). νₘₐₓ(KBr disc) 1772, 1623, 1559, and 1528cm⁻¹. δ_{H}(D₂O) 3.47 and 3.90 (2H,ABq,J 17.5Hz), 3.97(3H,s,), 4.29(2H,s), 5.35(1H,d,J 4.9Hz), 5.86(1H,d,J 4.9Hz), 6.09(1H,s), and 7.00(1H,s). [Mass spectrum : +ve ion (thioglycerol) MH⁺ (536)].

### EXAMPLE 10

### Sodium (6R,7R)-7-[2-(2-Aminothiazol-4-yl)-2-(Z)-pentenamido]-3-[((3RS-2-oxotetrahdrothien-yl)thio]ceph-3 -em-4-carboxylate.

### (a) Diphenylmethyl (6R,7R)-7-[2-(2-Aminothiazol-4-yl)-2-(Z)-pentenamido]-3-[((3RS)-2-oxotetrahydrothien-3 -yl)thio]ceph-3-em-4-carboxylate.

A stirred solution of 2-(2-Aminothiazol-4-yl)-(Z)-pentenoic acid (99mg) (see GB 2,173,194 A) in dimethylformamide (3ml) was cooled to -50°C under an atmosphere of argon. N,N-Diisopropylethylamine (86µl) and methanesulphonyl chloride (39µl) were added and the reaction stirred at -50°C for 30 min, before allowing to warm to -20°C. N,N-Diisopropylethylamine (86µl) was added, followed by a solution of diphenylmethyl (6R,7R)-7-amino-3-[((3RS)-2-tetrahydrothien-3-yl)thio]ceph-3-em-4-carboxylate (250mg) (see Example 6b) in dimethylformamide (2ml) and the reaction was stirred at 0°C for 15min. The reaction was poured into ethyl acetate and water. After separation the organic phase was washed with water (twice) and brine before drying over anhydrous magnesium sulphate. Purification was accomplished by chromatography on silica gel (Kielselgel) employing gradient elution (2:3 ethyl acetate/hexane rising to 3:2 ethyl acetate/hexane) gave the title compound a mixture of diastereomers which was isolated as a foam (23mg). νₘₐₓ(CH₂Cl₂) 3390, 1790, 1730, and 1690cm⁻¹. δ_{H}(CDCl₃) 1.06-1.21 (3H,m), 1.96-2.14(1H,m), 2.30-2.56(3H,m), 3.20-3.95(5H,m), 5.10(1H,d,J 4.7Hz), 5.76-5.92(3H,m,2H ex in D₂O), 6.34-6.45(1H,m), 6.90-6.96(1H,m), 7.26-7.53(10H,m), 7.99 and 8.10(1H,d+d,J 8.58Hz).
[mass spectrum : +ve ion (3-nitrobenzylalcohol/sodium acetate) MH⁺ (680) MNa⁺ (702)]

### (b) Sodium (6R,7R)-7-[2-(2-Aminothiazol-4-yl)-2-(Z)-pentenamido]-3-[((3RS)-2-oxotetrahydrothien-3-yl)-thio]ceph -3-em-4-carboxylate.

Diphenylmethyl (6R,7R)-7-[2-(2-aminothiazol-4-yl)-2-(Z)-pentenamido]-3-[((3RS)-2-oxotetrahydrothien-3-yl)-thio]ceph-3-em-4-carboxylate (203mg) was dissolved in cold trifluoroacetic acid (4ml) and the reaction stirred at 0°C for 15 min. The solvent was removed by evaporation in vacuo before evaporating solvent from the residue (three times). The residue was then dissolved in ethyl acetate/water and the pH adjusted to 6.3 with saturated aqueous sodium bicarbonate. After evaporating the aqueous phase to low volume purification was accomplished by chromatography on HP20SS resin, eluting with aqueous mixtures containing an increasing proportion of tetrahydrofuran. The product containing fractions were combined, evaporated to low volume, and freeze dried to give the title compound as a white solid (65mg). νₘₐₓ(KBr disc) 1762, 1664, 1609, and 1556cm⁻¹. δ_{H}(D₂O) 1.03(3H,t,J 7.4Hz) 2.14-2.19(3H,m), 2.54-2.68(1H,m), 3.33-3.61(3H,m), 5.23 and 5.24(1H,d+d,J 4.8Hz), 5.76(1H,d,J 4.8Hz), 6.33(1H,t,J 8.0Hz), and 6.46(1H,s).
[mass spectrum : +ve ion (thioglycerol) MH⁺ (535)].

### In Vitro Biological Data

### MIC (µg/ml)

| Organism | | |
|---|---|---|
| Example No. | E. coli (NCTC 1048) | S. aureus (Oxford) |
| 1 | ≤ 0.03 | 0.25 |
| 3 | 0.12 | 2.00 |
| 5 | 0.06 | 4.00 |
| 6 | 0.12 | 0.50 |
| 8(l) | 0.25 | - |
| 8(m) | 0.25 | - |
| 9 | 0.50 | 1.00 |
| 10 | 2.00 | 1.00 |

## Claims

1. A compound of formula (I) or a salt thereof: 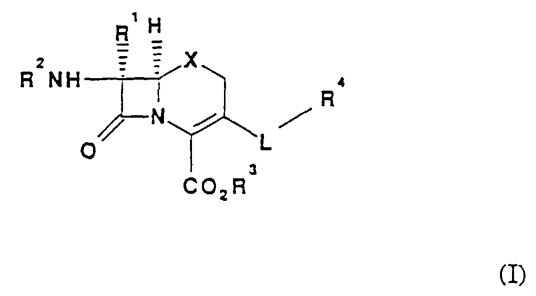 wherein
R¹ is hydrogen, methoxy or formamido;
R² is an acyl group;
CO₂R³ is a carboxy group or a carboxylate anion, or R³ is a readily removable carboxy protecting group;
L is -CH=,-(CH₂)ₙ where n is 0 or 1, -(CH₂)ₓ-Y-(CH₂)_{y}- where x and y are independently 0 or 1 and Y is sulphur or oxygen;
R⁴ is a γ- or δ-thiolactone or lactam ring optionally containing one or (where applicable) two endocyclic double bonds, which ring is optionally substituted at any carbon atom by alkyl, alkenyl, alkynyl, amino, acylamino dialkylamino, alkoxy, hydroxy, halogen, carboxy, C₁₋₆ alkoxycarbonyl, amido, di-(C₁₋₆)alkylamido, di-(C₁₋₆)alkylsulphonyl, heterocyclyl or aryl, which, in the case of more than one substituent, may be the same or different, or is optionally di-substituted at two adjacent carbon atoms which are available for substitution, to form an aromatic fused bicyclic system; and X is S, SO or SO₂.

2. A compound as claimed in claim 1 having the formula (Ia): 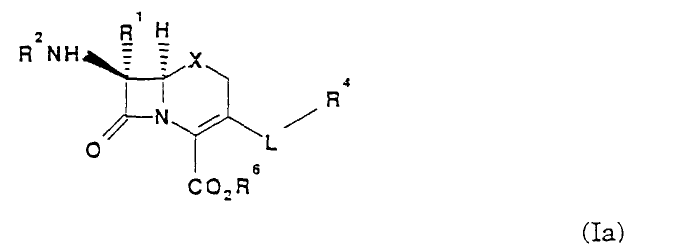 wherein R¹, R², L, R⁴ and X are as defined with respect to formula (I) in claim 1 and the group CO₂R⁶ is CO₂R³ where CO₂R³ is a carboxy group or a carboxylate anion, or a pharmaceutically acceptable salt or in vivo hydrolysable ester thereof.

3. A compound as claimed in claim 1 or claim 2 wherein R¹ is hydrogen.

4. A compound as claimed in claim 1,2 or 3 wherein R² is an acyl group of formula (a) to (f): 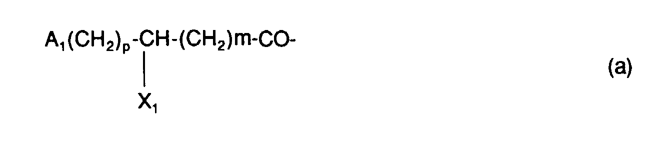
A₂CO- (b) 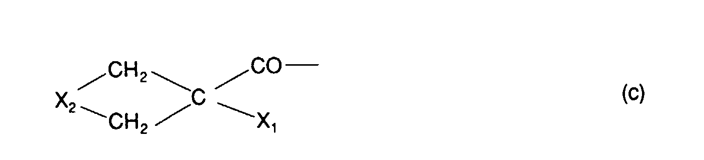
A₂-X₃-(CH₂)ₚ-CO- (d) 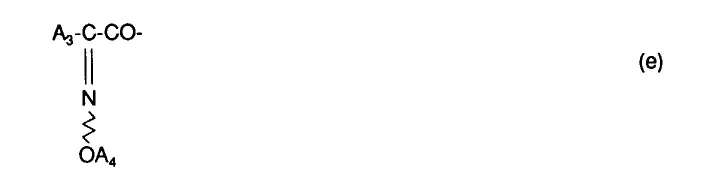 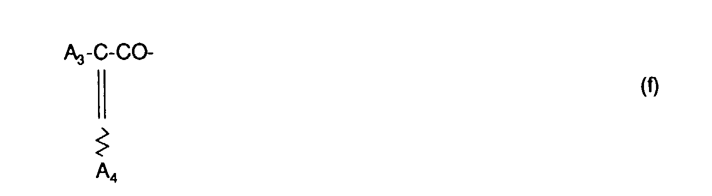
wherein p is 0, 1 or 2; m is 0, 1 or 2; A₁ is C₁₋₆ alkyl, substituted C₁₋₆ alkyl, C₃₋₆ cycloalkyl, cyclohexenyl, cyclohexadienyl, an aromatic or heteroaromatic group; X₁ is a hydrogen or halogen atom, a carboxylic acid, carboxylic ester, sulphonic acid, azido, tetrazolyl, hydroxy, acyloxy, amino, ureido, acylamino, heterocyclylamino, guanidino or acylureido group; A₂ is an aromatic or heteroaromatic group, a substituted alkyl group; or a substituted dithietane; X₂ is a -CH₂OCH₂-, -CH₂SCH₂- or alkylene group; X₃ is an oxygen or sulphur atom; A₃ is an aryl or heteroaryl group; and A₄ is hydrogen, C₁₋₆alkyl, C₃₋₈ cycloalkyl, C₃₋₈ cycloalkyl(C₁₋ ₆)alkyl, C₁₋₆ alkoxycarbonyl(C₁₋₆) alkyl, C₂₋₆ alkenyl, carboxy(C₁₋ ₆)alkyl, C₂₋₆ alkynyl, aryl or C₁₋₆alkyl substituted by up to three aryl groups.

5. A compound as claimed in claim 4 wherein A₁ is optionally substituted phenyl, X₁ is hydrogen or amino, A₂ is optionally substituted phenyl, X₃ is oxygen , A₃ is aminothiazolyl, aminothiadiazolyl or furyl, and A₄ is hydrogen, C₁₋₆ alkyl, or carboxy C₁₋₆ alkyl.

6. A compound as claimed in any one of claims 1 to 5 wherein L is - (CH₂)ₙ where n is 0, or L is -(CH₂)ₓ-Y-(CH₂)_{y} where Y is sulphur, x is 0 or 1 and y is 0.

7. A compound as claimed in any one of claims 1 to 6 wherein R⁴ is optionally substituted 2-oxodihydrothienyl, 2-oxotetrahydrothienyl, or 2-oxopyrrolidinyl.

8. A compound as claimed in any one of claims 1 to 7 wherein X is sulphur.

9. A compound selected from the group consisting of:
sodium (6R,7R)-7-[2-(2-Aminothiazol-4-yl)-2-(Z)-methoxyinfinoacetamido]-3-[2,5-dihydro-2-oxothien-4-yl)thiomethyl]ceph-3-em-4-carboxylate,
pivaloyloxymethyl (6R,7R)-7-[2-(2-Aminothiazol-4-yl)-2-(Z)-methoxyiminoacetamido]-3-[2,5-dihydro-2-oxothien-4-yl)thiomethyl]ceph-3-em-4-carboxylate,
sodium (6R,7R)-7-[2-(2-Aminothiazol-4-yl)-2-(Z)-methoxyiminoacetamido]-3-[N-methoxy-2-oxopyrrolidin-3-yl)thio]ceph-3-em-4-carboxylate,
pivaloyloxymethyl (6R,7R)-7-[2-(2-Aminothiazol-4-yl)-2-(Z)-methoxyiminoacetamido]-3-[N-methoxy-2-oxopyrrolidin-3-yl)thio]ceph-3-em-4-carboxylate,
sodium (6R,7R)-7-[2-(2-Aminothiazol-4-yl)-2-(Z)-methoxyiminoacetamido]-3-[N-methoxy-2-oxopyrrolidin-3-yl)thio]-ceph-3-em-4-carboxylate,
sodium (6R,7R)-7-[2-(2-aminothiazol-4-yl)-2-(Z)-methoxyiminoacetamido]-3-[2-oxotetrahydrothien-3-yl)thio]ceph-3-em-4-carboxylate,
pivaloyloxymethyl (6R,7R)-7-[2-(2-aminothiazol-4-yl)-2-(Z)-methoxyiminoacetamido]-3-[2-oxotetrahydrothien-3-yl)thio]ceph-3-em-4-carboxylate,
sodium (6R,7R)-7-[2-(2-aminothiazol-4-yl)-2-(Z)-methoxyiminoacetamido]-3-[(2-oxotetrahydrothien-5-yl)]ceph-3-em-4-carboxylate and
sodium (6R,7R)-7-[2-(2-Aminothiazol-4-yl)-2-(Z)-pentenamido-3-[(2-oxotetrahydrothien-3-yl)thio]ceph-3-em-4-carboxylate.

10. A process for the preparation of a compound of formula (I) as defined in any one of claims 1 to 9 which process comprises:
(a) treating a compound of formula (II) or a salt thereof: 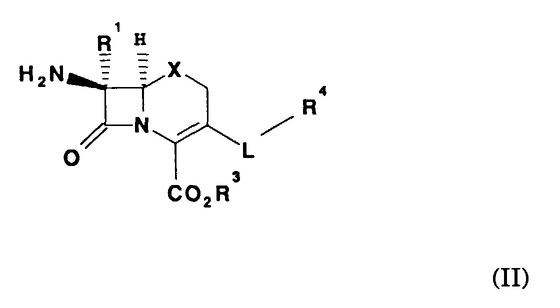 wherein R¹, R³, R⁴, X and L are as hereinbefore defined with respect to formula (I) in claim 1, wherein any reactive group may be protected, and wherein the amino group is optionally substituted with a group which permits acylation to take place, with an N-acylating derivative of an acid of formula (III):
R²OH (III)
wherein R² is as hereinbefore defined with respect to formula (I) in claim 1 and wherein any reactive group may be protected; or
(b) treating a compound of formula (IV): 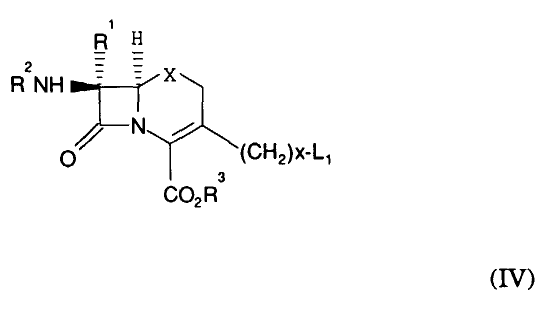 wherein X, R¹, R², x and R³ are as hereinbefore defined with respect to formula (1) in claim 1 and L₁ is a Y-group precursor (or a leaving group) with a compound of formula (V):
L₂-(CH₂)_{y}-R⁴ (V)
wherein L₂ is a leaving group (or a Y-group precursor) and R⁴ and y are as hereinbefore defined with respect to formula (1) in claim 1; or
(c) cyclising a compound of formula (VI): 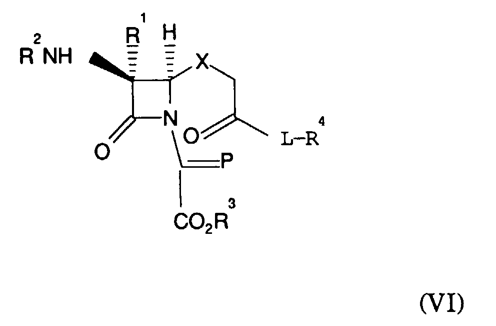 wherein X, R¹, R², R³ and R⁴ are as hereinbefore defined with respect to formula (I) in claim 1, L is -(CH₂)ₙ where n is 0 or 1, and P' is a phosphorus residue; or
(d) treating a compound of formula (VII): 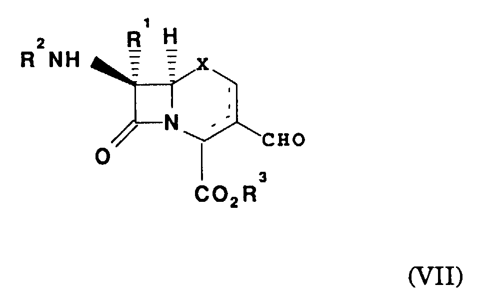 wherein R¹, R², R³ and X are as hereinbefore defined with respect to formula (I) in claim 1, and the dashed line represents a double bond in the 2- or 3- position of the cephalosporin nucleus, with a phosphorus ylid compound of formula (VIII):
P" = R⁴ (VIII)
wherein P" is the phosphorus residue and R⁴ is as hereinbefore defined with respect to formula (I) in claim 1;
and thereafter, if necessary or desired, carrying out one of the following steps:
i) removing any protecting groups;
ii) converting the group CO₂R³ to a different group CO₂R³;
iii) converting the group R² to a different group R²;
iv) converting the group X to a different group X;
v) reducing any endocyclic double bond within R⁴;
(vi) reducing an exocyclic double bond adjacent to R⁴;
vii) converting the product into a salt.

11. A compound of formula (II) or a salt thereof: 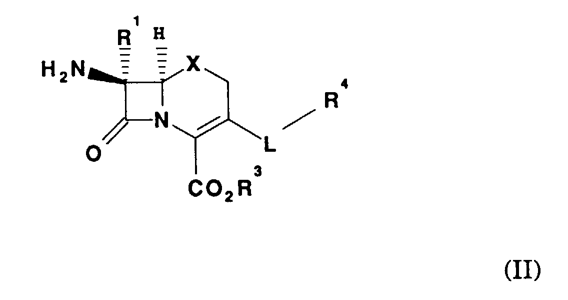 wherein R¹ R³, R⁴, L and X are as hereinbefore defined with respect to formula (I) in claim 1.

12. A compound selected from:
diphenylmethyl 6R,7R-7-Amino-3-[(2,5-dihydro-2-oxothien-4-yl)thiomethyl]ceph-3-em-4-carboxylate,
diphenylmethyl (6R,7R)-7-Amino-3-[N-methoxy-2-oxopyrrolidin-3-yl)thio]ceph-3-em-4-carboxylate,
diphenylmethyl (6R,7R)-7-Amino-3-[(N-methyl-2-oxopyrrolidin-3-yl)thio]ceph-3-em-4-carboxylate,
diphenylmethyl (6R,7R)-7-Amino-3-[(2-oxotetra-hydrothien-3-yl)thio]ceph-3-em-4-carboxylate,
t-Butyl (6R,7R)-7-Amino-3-[(2-oxotetrahydrothien-5-yl)thio]]ceph-3-em-4-carboxylate and
diphenylmethyl (6R,7R)-7-Amino-3-[(2,5-dihydro-2-oxothien-4-yl)thio]ceph-3-em-4-carboxylate.

13. A pharmaceutical composition comprising a compound of formula (Ia) as defined in claim 2 or a pharmaceutically acceptable salt or in vivo hydrolysable ester thereof, and a pharmaceutically acceptable carrier.

14. A pharmaceutical composition as claimed in claim 13 further comprising a β-lactamase inhibitor.

15. A compound of formula (Ia) or a pharmaceutically acceptable salt or in vivo hydrolysable ester thereof as defined in claim 2, for use as a therapeutic agent.

16. The use of a compound of formula (Ia) or a pharmaceutially acceptable salt or in vivo hydrolysable ester thereof, as defined in claim 2, for the manufacture of a medicament for the treatment of bacterial infections.

## Patentansprüche

1. Verbindung der Formel (I) oder ein Salz hiervon: 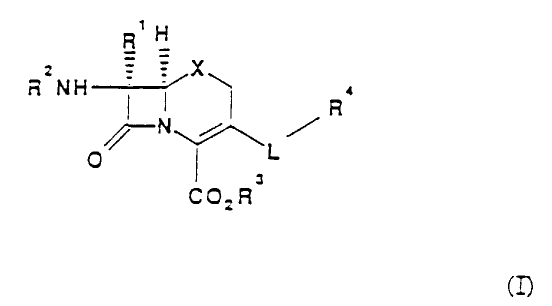 worin
R¹ für Wasserstoff, Methoxy oder Formamido steht,
R² eine Acylgruppe bedeutet,
CO₂R³ eine Carboxygruppe oder ein Carboxylatanion darstellt oder R³ einer bereitwillig entfernbaren Carboxyschutzgruppe entspricht,
L für -CH=, -(CH₂)ₙ- mit n gleich 0 oder 1, -(CH₂)ₓ-Y-(CH₂)_{y}- mit x und y unabhängig voneinander gleich 0 oder 1 und Y gleich Schwefel oder Sauerstoff, steht,
R⁴ einen γ- oder δ-Thiolacton- oder Lactamring, der gegebenenfalls eine oder (falls geeignet) zwei endocyclische Doppelbindungen enthält und gegebenenfalls an einem beliebigen Kohlenstoffatom durch Alkyl, Alkenyl, Alkinyl, Amino, Acylamino, Dialkylamino, Alkoxy, Hydroxy, Halogen, Carboxy, C₁₋₆-Alkoxycarbonyl, Amido, Di-(C₁₋₆)-alkylamido, Di-(C₁₋₆)-alkylsulfonyl, einen Heterocyclus oder Aryl (wobei im Falle von mehr als einem Substituenten die Substituenten gleich der verschieden sein können) substituiert ist oder gegebenenfalls an zwei benachbarten Kohlenstoffatomen, die für eine Substitution verfügbar sind, unter Bildung eines aromatischen ankondensierten bicyclischen Systems bisubstituiert ist, bedeutet und X S, SO oder SO₂ entspricht.

2. Verbindung nach Anspruch 1 mit folgender Formel (Ia): 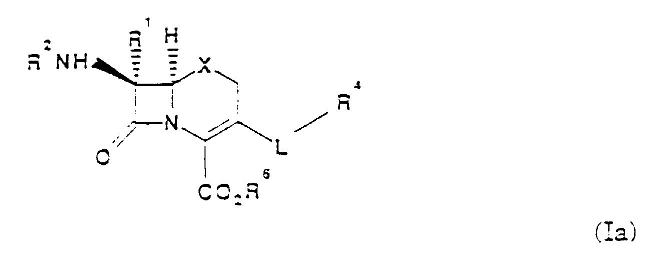 worin worin R¹, R², L, R⁴ und X die bei Formel (I) in Anspruch 1 angegebene Bedeutung besitzen und die Gruppe CO₂R⁶ CO₂R³ ist, wobei CO₂R³ für eine Carboxygruppe oder ein Carboxylatanion steht, oder ein pharmazeutisch akzeptables Salz oder ein in vivo-hydrolysierbarer Ester hiervon.

3. Verbindung nach Anspruch 1 oder 2, wobei R¹ für Wasserstoff steht.

4. Verbindung nach Anspruch 1, 2 oder 3, wobei R² für eine Acylgruppe der folgenden Formeln (a) bis (f) steht: 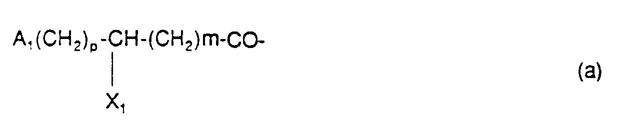
A₂CO- (b) 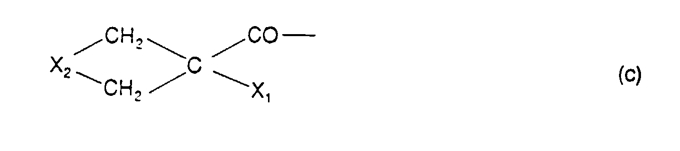
A₂-X₃-(CH₂)ₚ-CO- (d) 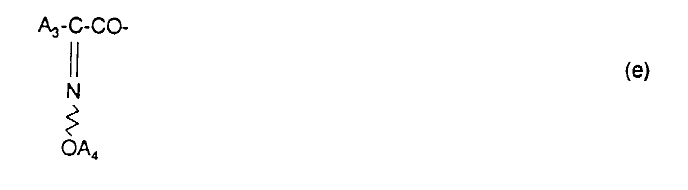 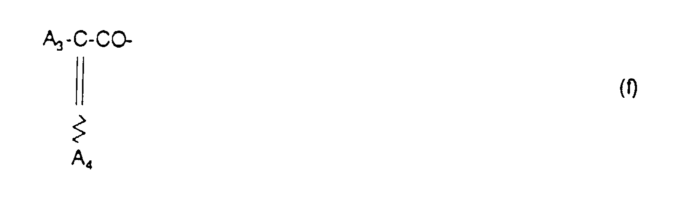
worin bedeuten:
p 0, 1 oder 2;
m 0, 1 oder 2;
A₁ eine C₁₋₆-Alkylgruppe, eine substituierte C₁₋₆-Alkylgruppe, eine C₃₋₆-Cycloalkylgruppe, eine Cyclohexenylgruppe, eine Cyclohexadienylgruppe, eine aromatische Gruppe oder eine heteroaromatische Gruppe;
X₁ ein Wasserstoff- oder Halogenatom, eine Carbonsäuregruppe, eine Carbonsäureestergruppe, eine Sulfonsäuregruppe, eine Azidogruppe, eine Tetrazolylgruppe, eine Hydroxygruppe, eine Acyloxygruppe, eine Aminogruppe, eine Ureidogruppe, eine Acylaminogruppe, eine heterocyclische Aminogruppe, eine Guanidinogruppe oder eine Acylureidogruppe;
A₂ eine aromatische oder heteroaromatische Gruppe, eine substituierte Alkylgruppe oder ein substituiertes Dithietan;
X₂ -CH₂OCH₂-, -CH₂SCH₂- oder eine Alkylengruppe;
X₃ ein Sauerstoff- oder Schwefelatom;
A₃ eine Aryl- oder Heteroarylgruppe und
A₄ Wasserstoff, C₁₋₆-Alkyl, C₃₋₈-Cycloalkyl, C₃₋₈-Cycloalkyl-(C₁₋₆)-alkyl, C₁₋₆-Alkoxycarbonyl-(C₁₋₆)-alkyl, C₂₋₆-Alkenyl, Carboxy-(C₁₋₆)-alkyl, C₂₋₆-Alkinyl, Aryl oder C₁₋₆-Alkyl, substituiert mit bis zu drei Arylgruppen.

5. Verbindung nach Anspruch 4, worin A₁ gegebenenfalls substituiertes Phenyl bedeutet, X₁ für Wasserstoff oder Amino steht, A₂ gegebenenfalls substituiertes Phenyl ist, X₃ Sauerstoff bedeutet, A₃ Aminothiazolyl, Aminothiadiazolyl oder Furyl darstellt und A₄ Wasserstoff, C₁₋₆-Alkyl oder Carboxy-C₁₋₆-alkyl entspricht.

6. Verbindung nach einem der Ansprüche 1 bis 5, wobei L für -(CH₂)ₙ- mit n gleich 0 steht oder L für -(CH₂)ₓ-Y-(CH₂)_{y} mit Y gleich Schwefel, x gleich 0 oder 1 und y gleich 0 steht.

7. Verbindung nach einem der Ansprüch 1 bis 6, wobei R⁴ für gegebenenfalls substituiertes 2-Oxodihydrothienyl, 2-Oxotetrahydrothienyl oder 2-Oxopyrrolidinyl steht.

8. Verbindung nach einem der Ansprüche 1 bis 7, wobei X für Schwefel steht.

9. Verbindung, ausgewählt aus der folgenden Gruppe:
Natrium-(6R,7R)-7-[2-(2-aminothiazol-4-yl)-2-(Z)-methoxyiminoacetamido]-3-[(2,5-dihydro-2-oxothien-4-yl)-thiomethyl]-ceph-3-em-4-carboxylat;
Pivaloyloxymethyl-(6R,7R)-7-[2-(2-aminothiazol-4-yl)-2-(Z)-methoxyiminoacetamido]-3-[(2,5-dihydro-2-oxothien-4-yl)-thiomethyl]-ceph-3-em-4-carboxylat;
Natrium-(6R,7R)-7-[2-(2-aminothiazol-4-yl)-2-(Z)-methoxyiminoacetamido]-3-[(N-methoxy-3-oxopyrrolidin-3-yl)-thio]-ceph-3-em-4-carboxylat;
Pivaloyloxymethyl-(6R,7R)-7-[2-(2-aminothiazol-4-yl)-2-(Z)-methoxyiminoacetamido]-3-[(N-methoxy-2-oxopyrrolidin-3-yl)-thio]-ceph-3-em-4-carboxylat;
Natrium-(6R,7R)-7-[2-(2-aminothiazol-4-yl)-2-(Z)-methoxyiminoacetamido]-3-[(N-methyl-2-oxopyrrolidin-3-yl)thio]-ceph-3-em-4-carboxylat;
Natrium-(6R,7R)-7-[2-(2-aminothiazol-4-yl)-2-(Z)-methoxyiminoacetamido]-3-[(2-oxotetrahydrothien-3-yl)-thio]-ceph-3-em-4-carboxylat;
Pivaloyloxymethyl-(6R,7R)-7-[2-(2-aminothiazol-4-yl)-2-(Z)-methoxyiminoacetamido]-3-[(2-oxotetrahydrothien-3-yl)-thio]-ceph-3-em-4-carboxylat;
Natrium-(6R,7R)-7-[2-(2-aminothiazol-4-yl)-2-(Z)-methoxyiminoacetamido]-3-[(2-oxotetrahydrothien-5-yl)]-ceph-3-em-4-carboxylat und
Natrium-(6R,7R)-7-[2-(2-aminothiazol-4-yl)-2-(Z)-pentenamido]-3-[(2-oxotetrahydrothien-3-yl)-thio]-ceph-3-em-4-carboxylat.

10. Verfahren zur Herstellung einer Verbidung der Formel (I) gemäß der Definition in einem der Ansprüche 1 bis 9 durch:
(a) Behandeln einer Verbindung der Formel (II) oder eines Salzes hiervon: 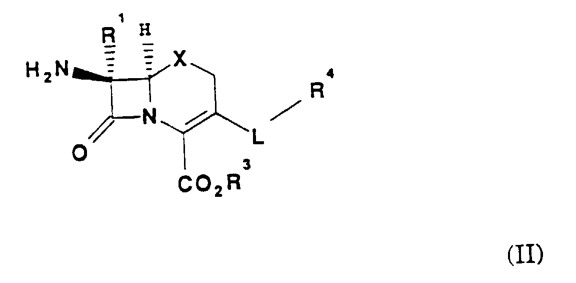 worin R¹, R³, R⁴, X und L die oben bei Formel (I) in Anspruch 1 angegebene Bedeutung besitzen, wobei jede beliebige reaktive Gruppe geschützt sein kann und wobei die Aminogruppe gegebenenfalls mit einer Gruppe substituiert ist, die das Ablaufen einer Acylierung erlaubt,
mit einem N-acylierenden Derivat einer Säure der Formel (III):
R²OH (III)
worin R² die oben bei Formel (I) in Anspruch 1 angegebene Bedeutung besitzt und wobei jede beliebige reaktive Gruppe geschützt sein kann; oder
(b) Behandeln einer Verbindung der Formel (IV): 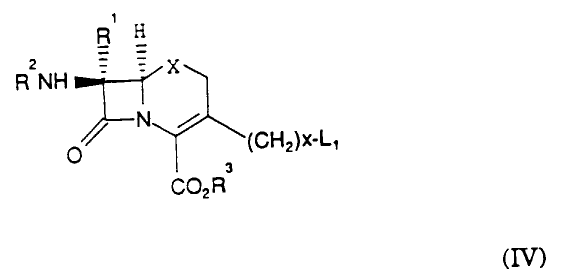 worin X, R¹, R², x und R³ die oben bei Formel (I) in Anspruch 1 angegebene Bedeutung besitzen und L₁ ein Y Gruppenvorläufer (oder eine Abgangsgruppe) ist,
mit einer Verbindung der Formel (V):
L₂-(CH₂)_{y}-R⁴ (V)
worin L₂ eine Abgangsgruppe (oder ein Y-Gruppenvorläufer ist) und R⁴ und y die oben bei Formel (I) in Anspruch 1 angegebene Bedeutung besitzen; oder
(c) Cyclisieren einer Verbindung der Formel (VI): 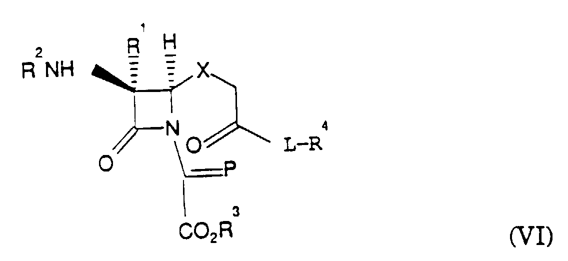 worin X, R¹, R², R³ und R⁴ die oben bei Formel (I) in Anspruch 1 angegebene Bedeutung besitzen, L für -(CH₂)ₙ- mit n gleich 0 oder 1 steht und P' ein Phosphorrest ist;
oder
(d) Behandeln einer Verbindung der Formel (VII): 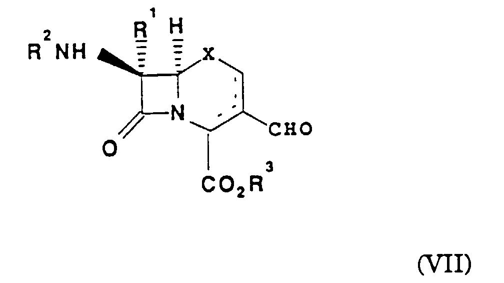 worin R¹, R², R³ und X die oben bei Formel (I) in Anspruch 1 angegebene Bedeutung besitzen und die gestrichelte Linie eine Doppelbindung in 2- oder 3-Stellung des Cephalosporinkerns bedeutet, mit einer Phosphorylidverbindung der Formel (VIII):
P" = R⁴ (VIII)
worin P" für den Phosphorrest steht und R⁴ die oben bei Formel (I) in Anspruch 1 angegebene Bedeutung besitzt;
und danach, wenn nötig oder gewünscht, durch Durchführen der folgenden Stufen:
i) Entfernen beliebiger Schutzgruppen;
ii) Umwandeln der Gruppe CO₂R³ in eine andere Gruppe CO₂R³;
iii) Umwandeln der Gruppe R² in eine andere Gruppe R²;
iv) Umwandeln der Gruppe X in eine andere Gruppe X;
v) Reduzieren einer beliebigen endocyclischen Doppelbindung in R⁴;
vi) Reduzieren einer exocyclischen, zu R⁴ benachbarten Doppelbindung;
vii) Umwandeln des Produkts in ein Salz.

11. Verbindung der Formel (II) oder ein Salz hiervon: 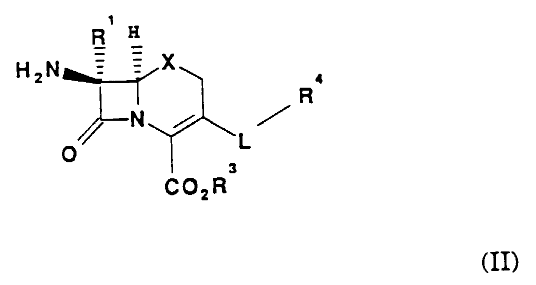 worin R¹, R³, R⁴ und L die oben bei Formel (I) in Anspruch 1 angegebene Bedeutung besitzen.

12. Verbindung, ausgewählt aus:
Diphenylmethyl-6R,7R-7-amino-3-[(2,5-dihydro-2-oxothien-4-yl)-thiomethyl]-ceph-3-em-4-carboxylat,
Diphenylmethyl-(6R,7R)-7-amino-3-[(N-methoxy-2-oxopyrrolidin-3-yl)-thio]-ceph-3-em-4-carboxylat,
Diphenylmethyl-(6R,7R)-7-amino-3-[(N-methyl-2-oxopyrrolidin-3-yl)-thio]-ceph-3-em-4-carboxylat,
Diphenylmethyl-(6R,7R)-7-amino-3-[(2-oxotetrahydrothien-3-yl)-thio]-ceph-3-em-4-carboxylat,
tert.-Butyl-(6R,7R)-7-amino-3-[(2-oxotetrahydrothien-5-yl)-thio]-ceph-3-em-4-carboxylat und
Diphenylmethyl-(6R,7R)-7-amino-3-[(2,5-dihydro-2-oxothien-4-yl)-thio]-ceph-3-em-4-carboxylat.

13. Pharmazeutische Zubereitung, die eine Verbindung der Formel (Ia) gemäß der Definition in Anspruch 2 oder ein pharmazeutisch akzeptables Salz oder einen in vivo-hydrolysierbaren Ester hiervon und einen pharmazeutisch akzeptablen Träger umfaßt.

14. Pharmazeutische Zubereitung nach Anspruch 13, die des weiteren einen β-Lactamasehemmer umfaßt.

15. Verbindung der Formel (Ia) oder ein pharmazeutisch akzeptables Salz oder ein in vivo-hydrolysierbarer Ester hiervon gemäß der Definition in Anspruch 2 zur Verwendung als therapeutisches Mittel.

16. Verwendung einer Verbindung der Formel (Ia) oder eines pharmazeutisch akzeptablen Salzes oder in vivo-hydrolyiserbaren Esters hiervon gemäß der Definition in Anspruch 2 zur Herstellung eines Medikaments zur Behandlung von bakteriellen Infektionen.

## Revendications

1. Composé de formule (I) ou sel de ce composé : 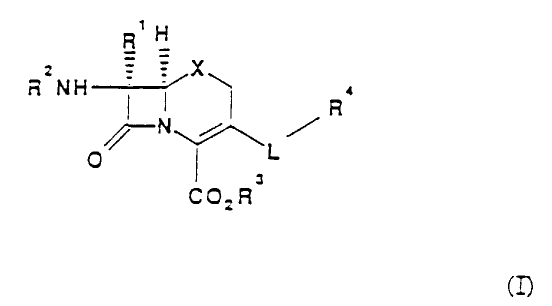 formule dans laquelle
R¹ est de l'hydrogène, un groupe méthoxy ou formamido ;
R² est un groupe acyle ;
CO₂R³ est un groupe carboxy ou un anion carboxylate, ou bien R³ est un groupe facile à éliminer protégeant la fonction carboxy ;
L est un groupe -CH=, -(CH₂)ₙ où n a la valeur 0 ou 1, -(CH₂)ₓ-Y-(CH₂)_{y}- où x et y ont, indépendamment, la valeur 0 ou 1, et Y est du soufre ou un oxygène ;
R⁴ est un noyau de γ- ou δ-thiolactone ou de lactame contenant une ou (lorsque cela est possible) deux doubles liaisons endocycliques, noyau qui est facultativement substitué au niveau de tout atome de carbone par un groupe alkyle, alcényle, alcynyle, amino, acylamino, dialkylamino, alkoxy, hydroxy, halogéno, carboxy, (alkoxy en C₁ à C₆)-carbonyle, amido, di-(alkyle en C₁ à C₆)amido, di-(alkyle en C₁ à C₆)sulfonyle, hétérocyclyle ou aryle, ces groupes pouvant être identiques ou différents au cas où il y a plus d'un substituant, ou qui est facultativement disubstitué au niveau de deux atomes adjacents de carbone qui sont disponibles pour une substitution, de manière à former un système bicyclique aromatique condensé ; et X représente S, SO ou SO₂.

2. Composé suivant la revendication 1, répondant à la formule (Ia) : 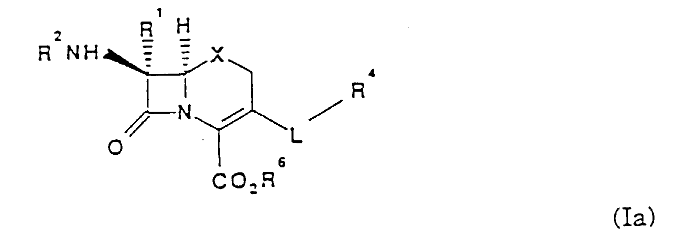 dans laquelle R¹, R², L, R⁴ et X sont tels que définis en ce qui concerne la formule (I) dans la revendication 1 et le groupe CO₂R⁶ est un groupe CO₂R³, ce groupe CO₂R³ étant un groupe carboxy ou un anion carboxylate, ou un sel acceptable du point de vue pharmaceutique ou un ester hydrolysable in vivo de ce composé.

3. Composé suivant la revendication 1 ou la revendication 2, dans lequel R¹ est de l'hydrogène.

4. Compmosé suivant la revendication 1, 2 ou 3, dans lequel R² est un groupe acyle de formule (a) à (f) : 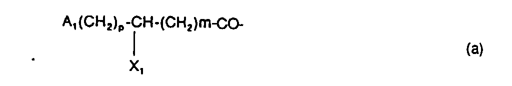
A₂CO- (b) 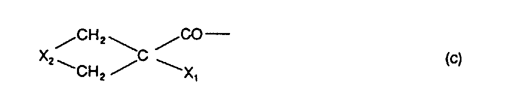
A₂-X₃-(CH₂)ₚ-CO- (d) 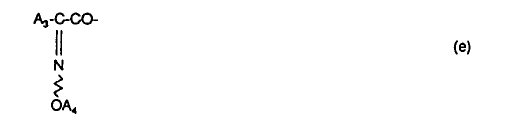 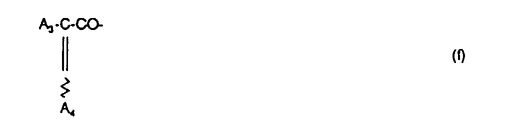
où p a la valeur 0 ou 1 ou 2 ; m a la valeur 0, 1 ou 2 ; A₁ est un groupe alkyle en C₁ à C₆, un groupe alkyle en C₁ à C₆ substitué, un groupe cycloalkyle en C₃ à C₆, cyclohexényle, cyclohexadiényle ou un groupe aromatique ou hétéroaromatique ; X₁ est un atome d'hydrogène ou d'halogène, un groupe acide carboxylique, ester carboxylique, acide sulfonique, azido, tétrazolyle, hydroxy, acyloxy, amino, uréiodo, acylamino, hétérocyclylamino, guanidino ou acyluréido ; A₂ est un groupe aromatique ou hétéroaromatique, un groupe alkyle substitué ; ou un dithiétane substitué ; X₂ est un groupe -CH₂OCH₂-, -CH₂SCH₂- ou un groupe alkylène ; X₃ est un atome d'oxygène ou de soufre ; A₃ est un groupe aryle ou hétéroaryle ; et A₄ est de l'hydrogène, un groupe alkyle en C₁ à C₆, cycloalkyle en C₃ à C₈, (cycloalkyle en C₃ à C₈)-(alkyle en C₁ à C₆), ((alkoxy en C₁ à C₆)carbonyle) (aîkyle en C₁ à C₆), alcényle en C₂ à C₆, carboxy-(alkyle en C₁ à C₆), alcynyle en C₂ à C₆, aryle ou alkyle en C₁ à C₆ portant jusqu'à trois substituants aryle.

5. Composé suivant la revendication 4, dans lequel A₁ est un groupe phényle facultativement substituté, X₁ est de l'hydrogène ou un groupe amino, A₂ est un groupe phényle facultativement substitué, X₃ est de l'oxygène, A₃ est un groupe aminothiazolyle, aminothiadiazolyle ou furyle, et A₄ est de l'hydrogène, un groupe alkyle en C₁ à C₆ ou un groupe carboxy-(alkyle en C₁ à C₆).

6. Composé suivant l'une quelconque des revendications 1 à 5, dans lequel L est un groupe (CH₂)ₙ où n est égal à 0, ou bien L est un groupe -(CH₂)ₓ-Y-(CH₂)_{y} dans lequel Y est un atome de soufre, x est égal à 0 ou 1 et y est égal à 0.

7. Composé suivant l'une quelconque des revendications 1 à 6, dans lequel R⁴ est un groupe 2-oxodihydrothiényle, 2-oxotétrahydrothiényle ou 2-oxopyrrolidinyle facultativement substitué.

8. Composé suivant l'une quelconque des revendications 1 à 7, dans lequel X est du soufre.

9. Composé choisi dans le groupe consistant en :
le (6R,7R)-7-[2-(2-aminothiazole-4-yl)-2-(Z)-méthoxyiminoacétamido]-3-[2,5-dihydro-2-oxothién-4-yl)thiométhyl]céph-3-ème-4-carboxylate de sodium,
le (6R,7R)-7-[2-(2-aminothiazole-4-yl)-2-(Z)-méthoxyiminoacétamido]-3-[2,5-dihydro-2-oxothién-4-yl)thiométhyl]céph-3-ème-4-carboxylate de pivaloyloxyméthyle,
le (6R,7R)-7-[2-(2-aminothiazole-4-yl)-2-(Z)-méthoxyiminoacétamido]-3-[N-méthoxy-2-oxopyrrolidine-3-yl)thiolcéph-3-ème-4-carboxylate de sodium,
le (6R,7R)-7-[2-(2-aminothiazole-4-yl)-2-(Z)-méthoxyiminoacétamido]-3-[N-méthoxy-2-oxopyrrolidine-3-yl)thiolcéph-3-ème-4-carboxylate de pivaloyloxyméthyle,
le (6R,7R)-7-[2-(2-aminothiazole-4-yl)-2-(Z)-méthoxyiminoacétamido]-3-[N-méthoxy-2-oxopyrrolidine-3-yl)thio]céph-3-ème-4-carboxylate de sodium,
le (6R,7R)-7-[2-(2-aminothiazole-4-yl)-2-(Z)-méthoxyiminoacétamido]-3-[2-oxotétrahydrothién-3-yl)thio]-céph-3-ème-4-carboxylate de sodium,
le (6R,7R)-7-[2-(2-aminothiazole-4-yl)-2-(Z)-méthoxyiminoacétamido]-3-[2-oxotétrahydrothién-3-yl)thio]-céph-3-ème-4-carboxylate de pivaloyloxyméthyle,
le (6R,7R)-7-[2-(2-aminothiazole-4-yl)-2-(Z)-méthoxyiminoacétamido]-3-[(2-oxotétrahydrothién-5-yl)]céph-3-ème-4-carboxylate de sodium, et
le (6R,7R)-7-[2-(2-aminothiazole-4-yl)-2-(Z)-penténamido-3-[(2-oxotétrahydrothién-3-yl)thiolcéph-3-ème-4-carboxylate de sodium.

10. Procédé de production d'un composé de formule (I) tel que défini dans l'une quelconque des revendications 1 à 9, procédé qui comprend :
(a) le traitement d'un composé de formule (II) ou d'un sel de ce composé : 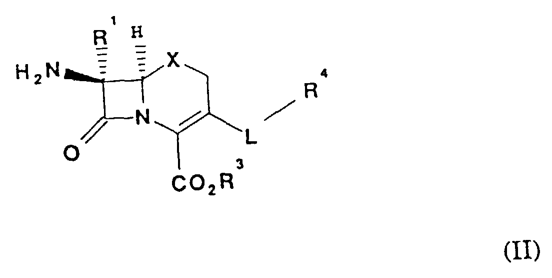 formule dans laquelle R¹, R³, R⁴, X et L sont tels que définis ci-dessus en ce qui concerne la formule (I) dans la revendication 1, dans laquelle tout groupe réactif peut être protégé, et dans laquelle le groupe amino est facultativement substitué avec un groupoe qui permet à une acylation de se produire, avec un dérivé N-acylant d'un acide de formule (III) :
R²OH (III)
dans laquelle R² est tel que défini ci-dessus en ce qui concerne la formule (I) dans la revendication 1 et dans laquelle tout groupe réactif peut être protégé ; ou bien
(b) le traitement d'un composé de formule (IV) ; 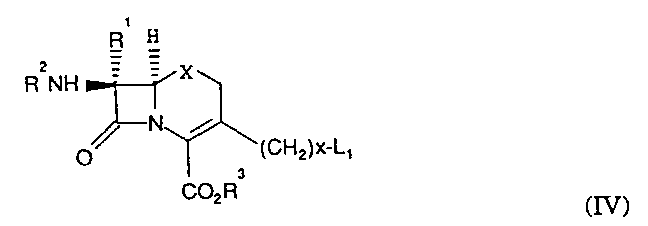 dans laquelle X, R¹, R², x et R³ sont tels que définis ci-dessus en ce qui concerne la formule (1) dans la revendication et L₁ est un précurseur du groupe Y (ou un groupe partant), avec un compossé de formule (V) :
L₂-(CH₂)_{y}-R⁴ (V)
dans laquelle L₂ est un groupe partant (ou un précurseur du groupe Y) et R⁴ et y sont tels que définis ci-dessus en ce qui concerne la formule (I) dans la revendication 1 ; ou bien
(c) la cyclisation d'un composé de formule (VI) : 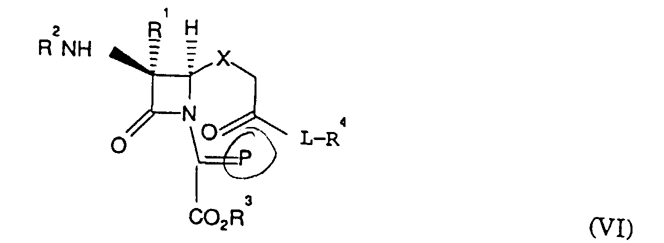 dans laquelle X, R¹, R², R³ et R⁴ sont tels que définis ci-dessus en ce qui concerne la formule (I) dans la revendication 1, L est un groupe -(CH₂)ₙ dans lequel n a la valeur 0 ou 1 et P' est un résidu phosphoré ; ou bien
(d) le traitement d'un composé de formule (VII) : 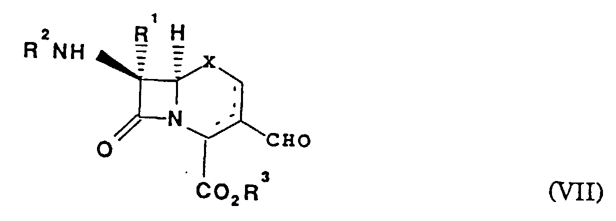 dans laquelle R¹, R², R³ et X sont tels que définis ci-dessus en ce qui concerne la formule (I) dans la revendication 1, et la ligne en tiretés représente une double liaison dans la position 2 ou 3 du noyau de céphalosporine, avec un ylide phosphoré de formule (VIII) :
P" = R⁴ (VIII)
dans laquelle P" est le résidu phosphoré et R⁴ est tel que défini ci-dessus en ce qui concerne la formule (I) dans la revendication 1 ;
puis, si c'est nécessaire ou si on le souhaite, conduite de l'une des étapes suivantes :
i) élimination de tous groupes protecteurs ;
il) conversion du groupe CO₂R³ en un groupe CO₂R³ différent ;
iii) conversion du groupe R² en un groupe R² différent ;
iv) conversion du groupe X en un groupe X différent ;
v) réduction de toute double liaison endocyclique existant dans R⁴ ;
vi) réduction d'une double liaison exocyclique adjacente à R⁴ ;
vii) conversion du produit en un sel.

11. Composé de formule (II) ou un sel de ce composé : 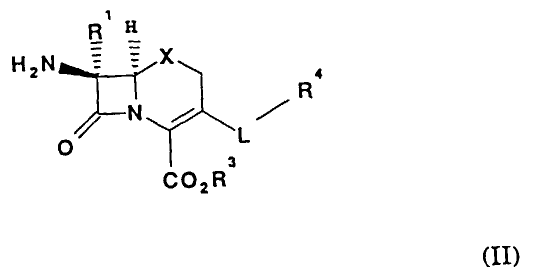 formule dans laquelle R¹, R³, R⁴, L et X sont tels que définis ci-dessus en ce qui concerne la formule (I) dans la revendication 1.

12. Composé choisi entre :
le 6R,7R-7-amino-3-[(2,5-dihydro-2-oxothién-4-yl)thiométhyl]céph-3-ème-4-carboxylate de diphénylméthyle,
le (6R,7R)-7-amino-3-[N-méthoxy-2-oxopyrrolidine-3-yl)thio]céph-3-ème-4-carboxylate de diphénylméthyle,
le(6R,7R)-7-amino-3-[N-méthyl-2-oxopyrrolidine-3-yl)thio]céph-3-ème-4-carboxylate de diphénylméthyle,
le (6R,7R)-7-amino-3-[(2-oxotétrahydrothién-3-yl)thio]céph-3-ème-4-carboxylate de diphénylméthyle,
le (6R,7R)-7-amino-3-[(2-oxotétrahydrothién-5-yl)thio]céph-3-ème-4-carboxylate de tertio-butyle, et
le(6R,7R)-7-amino-3-[(2,5-dihydro-2-oxothién-4-yl)thio]céph-3-ème-4-carboxylate de diphénylméthyle.

13. Composition pharmaceutique, comprenant un composé de formule (Ia) tel que défini dans la revendication 2 ou un sel pharmaceutiquement acceptable ou un ester hydrolysable in vivo de ce composé, et un support acceptable du point de vue pharmaceutique.

14. Composition pharmaceutique suivant la revendication 13, comprenant en outre un inhibiteur de β-lactamase.

15. Composé de formule (Ia) ou sel pharmaceutiquement acceptable ou ester hydrolysable in vivo de ce composé tel que défini dans la revendication 2, destiné à être utilisé comme agent thérapeutique.

16. Utilisation d'un composé de formule (Ia) ou d'un sel pharcameutiquement acceptable ou d'un ester hydrolysable in vivo de ce composé, tel que défini dans la revendication 2, pour la préparation d'un médicament destiné au traitement d'infections bactériennes.
